# EUROPEAN PATENT SPECIFICATION

(11) **EP 4 138 883 B1**
(45) Date of publication and mention of the grant of the patent: **10.09.2025**
(21) Application number: 21717923.3
(22) Date of filing: 16.04.2021
(51) Int. Cl.: C07K 14/31, C12N 9/50, A61K 38/00, A61K 38/47

(54) **A CHIMERIC ENDOLYSIN POLYPEPTIDE**
CHIMÄRES ENDOLYSIN-POLYPEPTID
POLYPEPTIDE ENDOLYSINE CHIMÉRIQUE

(30) Priority: 20.04.2020 EP 20170432
(43) Date of publication of application: 01.03.2023
(73) Proprietor: Micreos Human Health B.V., 2585 EV Den Haag (NL)
(72) Inventor: EICHENSEHER, Fritz, 2585 EV Den Haag (NL); SCHMELCHER, Mathias, 2585 EV Den Haag (NL); RÖHRIG, Christian Alexander, 2585 EV Den Haag (NL)
(74) Representative: Nederlandsch Octrooibureau
(86) International application number: PCT/EP2021/059849
(87) International publication number: WO 2021/213898

(56) References cited:
- WO-A1-2012/150858
- WO-A1-2015/005787
- WO-A1-2018/091707
- WO-A2-2009/024327
- WO-A2-2010/011960
- US-A1- 2019 374 621
- US-B2- 10 357 547
- US-B2- 11 439 693
- CHANG YOONJEE ET AL: "Characterization of a novel cell wall binding domain-containingStaphylococcus aureusendolysin LysSA97", APPLIED MICROBIOLOGY AND BIOTECHNOLOGY, SPRINGER BERLIN HEIDELBERG, BERLIN/HEIDELBERG, vol. 101, no. 1, 6 August 2016 (2016-08-06), pages 147 - 158, XP036123001, ISSN: 0175-7598, [retrieved on 20160806], DOI: 10.1007/S00253-016-7747-6
- ANONYMOUS: "Endolysin/amidase/endopeptidase - Staphylococcus phage Quidividi | UniProtKB | UniProt", 7 November 2018 (2018-11-07), pages 1 - 3, XP093077240, Retrieved from the Internet <URL:https://www.uniprot.org/uniprotkb/A0A345AP02/entry> [retrieved on 20230830]
- MELO LUÍS D. ET AL: "Isolation and characterization of a new Staphylococcus epidermidis broad-spectrum bacteriophage", JOURNAL OF GENERAL VIROLOGY, vol. 95, no. 2, 1 February 2014 (2014-02-01), pages 506 - 515, XP093194702, ISSN: 0022-1317, Retrieved from the Internet <URL:https://doi.org/10.1099/vir.0.060590-0> DOI: 10.1099/vir.0.060590-0
- DATABASE GenBank [online] NCBI; 25 February 2014 (2014-02-25), ANONYMOUS: "endolysin [Staphylococcus phage phiIBB-SEP1]", XP093194714, retrieved from https://www.ncbi.nlm.nih.gov/protein/AGR48314 Database accession no. AGR48314.1
- "Advances in Virus Research", vol. 83, 1 January 2012, ACADEMIC PRESS, US, ISSN: 0065-3527, article DANIEL C. NELSON ET AL: "Endolysins as Antimicrobials", pages: 299 - 365, XP055220045, DOI: 10.1016/B978-0-12-394438-2.00007-4
- HUGO OLIVEIRA ET AL: "Staphylococci phages display vast genomic diversity and evolutionary relationships", BMC GENOMICS, BIOMED CENTRAL LTD, LONDON, UK, vol. 20, no. 1, 9 May 2019 (2019-05-09), pages 1 - 14, XP021271804, DOI: 10.1186/S12864-019-5647-8

## Description

### Field of the invention

The invention relates to the field of medicine, specifically to the field of treatment of conditions associated with *Staphylococcus* infection. The invention relates to a novel endolysin polypeptide specifically targeting a bacterial *Staphylococcus* cell. The invention further relates to said endolysin polypeptide for medical use, preferably for treating an individual suffering from a condition associated with *Staphylococcus* infection.

### Background of the invention

The Gram-positive bacteria of the genus *Staphylococci* are a major concern in human health care, mostly due to the increasing prevalence of antibiotic-resistance. They can infect virtually any tissue, including the skin, soft tissues, bones, the heart, the lung and even the brain (Tong, 2015). Additionally, biofilms can serve as centres of infections for systemic infections and sepsis, for example on prosthetic devices and catheters. Among the most prevalent bacterial species are the coagulase-positive *S*. *aureus* and the coagulase-negative *S. epidermidis.* Infections with *Staphylococci* are often difficult to treat due to high levels of antibiotics resistance and the physically restricted access to the centres of infection. There is a high demand for novel drugs, active against *Staphylococci.*

Peptidoglycan hydrolases (PGHs) can cleave specific bonds within the peptidoglycan (PG) network of bacteria and have been shown to be active against biofilms. Their high lytic activity makes PGHs potent anti-staphylococcal agents. Endolysins are highly specific, phage-derived PGHs, active against both drug-sensitive and resistant bacteria (Schmelcher et al. 2012). As potential alternatives to antibiotics, they have undergone investigations *in vitro, in vivo* and are under trial in several clinical studies (Kashani et al. 2017). PGHs of *Staphylococci* regularly display a domain-like architecture, consisting of enzymatically active domains (EADs) and cell-wall-binding domains (CBDs). The high specificity of staphylococcal PGHs may be attributed to their CBDs, which regularly feature an SH3b-fold. The structures of staphylococcal endolysin SH3b domains have been solved and display great homology to the bacteriocins lysostaphin (LST) and ALE1, suggesting a common recognition site in the PG.

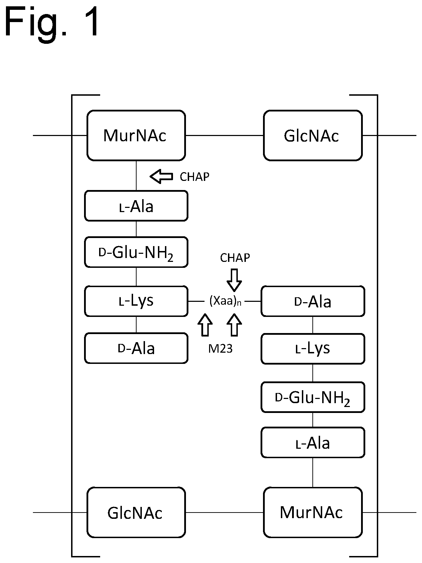

The EADs are more diverse and can be grouped according to their structure and cleavage site within the PG. Cysteine, histidine dependent amidohydrolase/peptidase (CHAP) domains are frequently found in staphylolytic endolysins, for example in phage Twort or phage K (Korndörfer et al. 2006). Depending on the CHAP domain present, cleavage can occur at different locations in the PG, including the amide bond of the sugar backbone to the stem peptide and the link of the stem peptide to the peptide cross bridge (Figure 1). Herein, the amidohydrolase/peptidase activity of a CHAP domain is referred to as CHAP activity. M23 domains, have only been found in one endolysin (phage 2638), but are also present in the staphylococcal bacteriocins LST and its homologue ALE1. The M23 domains of LST and ALE1 cleave the pentaglycine cross-bridge, which connects adjacent stem peptides in the PG of *S*. *aureus,* whereas the M23 domain of phage 2638 cuts between the peptide bridge and the stem peptide (Figure 1) (Gründling et al. 2006; Schmelcher et al. 2015 JAC). Herein, the peptidase activity of an M23 domain is referred to as M23 peptidase activity or M23 activity.

Previously, effectivity has been assessed positively in milk for combinations of separate PGHs against *S*. *aureus* with orthogonal cut sites in the PG (Verbree et al. 2018). This may be because cleavage of one bond within the PG network may result in better accessibility of different bonds within the three-dimensional PG network. WO2018091707A1, WO2012150858A1 and WO2010011960A2 each discloses chimeric endolysins having lytic activity against *S*. *aureus* and comprising the domains CHAP, M23 endopeptidase and cell wall binding of endolysins originated from different phages. Melo Luis et al. (2014) discloses the isolation and characterization of the phage SEP1 having lytic activity against *S. epidermidis.*

Altogether, especially for systemic infections and sepsis and for prosthetic devices and catheters, there is a need for a single endolysin polypeptide with improved characteristics on antimicrobial activity and stability against both coagulase positive and coagulase negative *Staphylococcus* species, such as *S*. *aureus* and S *epidermidis.*

### Description of the invention

The inventors have established that a combination of an M23 endopeptidase and a CHAP domain on a single chimeric endolysin polypeptide provides desired improved activity and stability, notably in human serum.

Accordingly, in a first aspect there is provided for an endolysin polypeptide that has lytic activity for *Staphylococcus,* wherein the endolysin polypeptide has enhanced specific activity for *Staphylococcus epidermidis* and/or enhanced stability in human serum at 37°C compared to the endolysin with the amino acid sequence as set forward in SEQ ID NO: 4, and wherein the amino acid sequence of the endolysin polypeptide has at least 90% sequence identity with SEQ ID NO: 35.

An overview of the sequences herein is provided in Table 1.

The endolysin polypeptide is herein interchangeably referred to as the endolysin polypeptide as disclosed herein, the endolysin as disclosed herein, the endolysin polypeptide and the endolysin.

In an embodiment, the endolysin polypeptide has lytic activity for both coagulase-positive as coagulase-negative species of *Staphylococcus,* especially for both *Staphylococcus aureus* and *Staphylococcus epidermidis.*

In the disclosure herein, the polypeptide domain with cysteine, histidine-dependent aminopeptidase (CHAP) activity may be CHAP-SEP (as set forward in SEQ ID NO: 11). Other CHAP domains known to the person skilled in the art, such as, CHAP-Tw (as set forward in SEQ ID NO: 1), CHAP-K (as set forward in SEQ ID NO: 7), CHAP-GH15 (as set forward in SEQ ID NO: 12), TCHAP-K (as set forward in SEQ ID NO: 13) and TCHAP-TW (as set forward in SEQ ID NO: 15) are also disclosed herein but not encompassed by the wording of the claims.

In the disclosure herein, the polypeptide domain with M23 peptidase activity may be M23-LST (as set forward in SEQ ID NO: 2). Other M23 domains known to the person skilled in the art, such as, M23-2638 (as set forward in SEQ ID NO: 8), and M23-ALE1 (as set forward in SEQ ID NO: 14) are also disclosed herein but not encompassed by the wording of the claims.

In the disclosure herein, the polypeptide domain with cell wall binding activity may be SH3b-LST (as set forward in SEQ ID NO: 9). Other cell wall binding- or SH3b domains known to the person skilled in the art, such as, SH3b-ALE1 (as set forward in SEQ ID NO: 10) and SH3b-2638 (as set forward in SEQ ID NO: 3) are also disclosed herein but not encompassed by the wording of the claims.

In the disclosure herein, the endolysin polypeptide may comprise:
- a polypeptide domain with CHAP activity, wherein the amino acid sequence of the polypeptide domain has at least 90, 91, 92, 93, 94, 95, 96, 97, 98, 99% or 100% sequence identity with SEQ ID NO: 11;
- a polypeptide domain with M23 peptidase activity, wherein the amino acid sequence of the polypeptide domain has at least 90, 91, 92, 93, 94, 95, 96, 97, 98, 99% or 100% sequence identity with SEQ ID NO: 2;
- a polypeptide domain with cell wall binding activity, wherein the amino acid sequence of the polypeptide domain has at least 90, 91, 92, 93, 94, 95, 96, 97, 98, 99% or 100% sequence identity with SEQ ID NO: 9;
wherein the endolysin polypeptide has enhanced specific activity for *Staphylococcus epidermidis* and/or enhanced stability in human serum at 37°C compared to the endolysin with the amino acid sequence as set forward in SEQ ID NO: 4.

In the disclosure herein, the polypeptide domains are in the orientation: - CHAP - M23 peptidase - cell wall binding domain.

In the disclosure herein, the endolysin polypeptide may comprise polypeptide domains having at least 90% sequence identity with: SEQ ID NO: 11, 2, and 9.

The person skilled in the art will comprehend that the disclosed domains are separated by a linker. Disclosed herein but not encompassed by the wording of the claims is a peptide linker or oligopeptide linker such as a linker selected from the group consisting of the linkers having an amino acid sequence of at least 80, 81, 82, 83, 84, 85, 86, 87, 88, 89 ,90, 91, 92, 93, 94, 95, 96, 97, 98, 99% or 100% sequence identity with a sequence selected from the group consisting of SEQ ID NO:s 16 - 21. The preferred disclosed linker has an amino acid sequence with at least 90, 91, 92, 93, 94, 95, 96, 97, 98, 99% or 100% sequence identity with SEQ ID NO: 22.

Accordingly, in the disclosure herein, the endolysin polypeptide has at least two domains that are separated by a linker, such linker having acid sequence with at least 90, 91, 92, 93, 94, 95, 96, 97, 98, 99% or 100% sequence identity with SEQ ID NO: 22.

In the disclosure herein, the endolysin polypeptide has at least 90, 91, 92, 93, 94, 95, 96, 97, 98, 99% or 100% sequence identity with SEQ ID NO: 35. Further provided is a polynucleotide encoding an endolysin polypeptide as disclosed herein. Said polynucleotide is herein referred to as a polynucleotide as disclosed herein. Also provided is a nucleic acid construct comprising a polynucleotide as disclosed herein. Said nucleic acid construct is herein referred to as a nucleic acid construct as disclosed herein. Also provided is an expression vector comprising a nucleic acid construct as disclosed herein. Said expression vector is herein referred to as an expression vector as disclosed herein. An expression vector as disclosed herein may be a recombinant expression vector. Such vector may constitute a plasmid, a cosmid, a bacteriophage or a virus, or a part thereof, which is transformed by introducing a nucleic acid construct or a polynucleotide as disclosed herein. Such transformation vectors specific to the host organism to be transformed are well known to those skilled in the art and widely described in the literature. In order to produce a polynucleotide or endolysin polypeptide as disclosed herein in a host, a process for the transformation of a host organism, and integration of a polynucleotide, nucleic acid construct or expression vector as disclosed herein may be appropriate. Such transformation may be carried out by any suitable known means which have been widely described in the specialist literature and are well-known to the person skilled in the art. Also provided is a host cell comprising a polynucleotide as disclosed herein, a nucleic acid construct as disclosed herein or an expression construct as disclosed herein. Said host cell is herein referred to as a host cell as disclosed herein. A host cell as disclosed herein may be any microbial, prokaryotic or eukaryotic, cell which is suitable for expression of the endolysin polypeptide as disclosed herein. Preferably, said cell is an *E*. *coli,* such as *E*. *coli* XL1 blue MRF, *E. coli* BL21 (DE3).

Further disclosed is a method for the production of an endolysin polypeptide as disclosed herein comprising:
- culturing a host cell as disclosed herein under conditions conducive to the production of the endolysin polypeptide,
- optionally isolating and purifying the endolysin polypeptide from the culture broth, and
- optionally freeze-drying the endolysin polypeptide.

Preferably, an *E*. *coli* is used in the method for producing an endolysin polypeptide as disclosed herein. Preferably, an *E*. *coli* XL1blueMRF or *E*. *coli* BL21-Gold(DE3) is used in step i). Preferably, when a His-tag is used, in the step of isolation and purification, IMAC and Econo-Pac Chromatography columns (Biorad) packed with 5mL low density Nickel chelating agarose beads (ABT beads) in combination with gravity flow are used to purify an endolysin polypeptide as disclosed herein. The eluted polypeptide can be dialyzed for 2, 4, and 12 hours against 3 x 1I lyophilization buffer, said buffer preferably comprising 50 mM phosphate, 500mM sucrose, 200mM mannitol, 0.005% polysorbate20, pH 7.4. In an embodiment, no His-tag is used; preferably no tag at all is used.

Lyophilisation and reconstitution are preferably construed as dehydration by freeze-drying and subsequent reconstitution of the sample by adding water. Preferably, lyophilisation and reconstitution is performed by dialyzing against 3 changes of 300 ml lyophilization buffer (50 mM phosphate or Tris, 500 mM sucrose, 200 mM mannitol, pH 7.4) aliquot and freezing in the gaseous phase of liquid nitrogen. Freeze-drying is preferably performed under standard conditions, preferably at -40°C and vacuum at 75 mTorr for 60 minutes, followed by increasing the temperature during 5 hours to -10°C and another 60 minutes at -10°C at the same vacuum levels. As a final step, the temperature is preferably increased to 25°C during 10 hours. Samples are preferably reconstituted by the addition of water.

Further disclosed is a method for purifying an endolysin polypeptide as disclosed herein with enhanced activity comprising dialysis of an endolysin polypeptide as disclosed herein, said dialysis comprising the steps of:
i) dialysis against a buffer comprising a chelating compound, and
ii) dialysis against a divalent metal ion-containing buffer, preferably a divalent metal ion selected from the group consisting of Co²⁺, Cu²⁺, Mg²⁺, Ca²⁺, Mn²⁺ and Zn²⁺.

A "chelating compound" is defined herein as a compound that binds a metal ion. Well known chelating compounds are ethylene diammine tetraacetic acid (EDTA) and ethylene glycol tetraacetic acid (EGTA). Preferably EDTA is used in step i) of the method of the disclosure.

Preferably, the divalent metal ion of step ii) is selected from the group consisting Mn²⁺, Co²⁺, Cu²⁺, more preferably, said divalent metal ion is selected from the group consisting of Mn²⁺ and Co²⁺, even more preferably said divalent metal ion is Mn²⁺.

It has been demonstrated that substituting a divalent metal ion by any of the above defined resulted in an increase of a lytic activity of Ply2638 of 2 - 2.5 fold. Lytic activity was assessed spectrophotometrically as described herein. Preferably, the method leads to an increase in a lytic activity of at least 1.1, 1.2, 1.3, 1.4, 1.5, 1.6, 1.7, 1.8, 1.9 or 2 fold as compared to an untreated polypeptide. Even more preferably, the method leads to an increase in a lytic activity of at least 2.5 fold. Preferably, the treated polypeptide exhibits a 0.7, 0.8, 0.9, 1.0, 1.1, 1.2, 1.3, 1.4, 1.5, 1.6, 1.7, 1.8, 1.9 to 2 fold increase in lytic activity as compared to the untreated polypeptide with the amino acid sequence as set forward in SEQ ID NO: 5 or SEQ ID NO: 6.

Further provided is a composition comprising an endolysin polypeptide as disclosed herein or a polynucleotide as disclosed herein, or a nucleic acid construct as disclosed herein, or an expression construct as disclosed herein, or a host cell as disclosed herein. Such composition as disclosed herein may comprise a mixture of different polynucleotides, and/or nucleic acid constructs and/or endolysin polypeptides and/or vectors and/or cells as disclosed herein or as obtainable by a method as disclosed herein. A composition as disclosed herein may further comprise a pharmaceutically acceptable excipient. Such composition is herein referred to as a pharmaceutical composition and is preferably for use as a medicine or as a medicament. Preferably the medicament is used in the treatment of infectious diseases, preferably infection with a *Staphylococcus.*

Accordingly, further provided is a pharmaceutical composition comprising an endolysin polypeptide as disclosed herein, a polynucleotide as disclosed herein, a nucleic acid construct as disclosed herein, an expression construct as disclosed herein, and/or a host cell as disclosed herein; said pharmaceutical composition further comprising a pharmaceutically acceptable excipient.

A composition or a pharmaceutical composition as disclosed herein may further comprise one or more additional active ingredients. Active preferably defined as showing a lytic activity as defined elsewhere herein. Preferably, said one or more additional active ingredients are selected from the group consisting of a bacteriophage or phage, a phage endolysin derived from such phage and an antibiotic. A phage encompassed herein can be any phage known in literature. Preferably, such phage is, but is not limited, from a family of the list consisting of *Myoviridae, Siphoviridae* and *Podoviridae.* Such phage may also be from a family of the list consisting of *Tectiviridae, Corticoviridae, Lipothrixviridae, Plasmaviridae, Rudiviridae, Fuselloviridae, Inoviridae, Microviridae, Leviviridae* and *Cystoviridae.* Within the context of the invention, a combination of active ingredients as defined herein can be administered sequentially or simultaneously. A composition as defined herein may be in the liquid, solid or semi-liquid or semi-solid form.

A composition of a pharmaceutical composition as disclosed herein may be used to treat animals, including humans, infected with *Staphylococcus* species as defined herein. Any suitable route of administration can be used to administer said composition including but not limited to: oral, aerosol or other device for delivery to the lungs, nasal spray, intravenous, intramuscular, intraperitoneal, intrathecal, vaginal, rectal, topical, lumbar puncture, intrathecal, and direct application to the brain and/or meninges.

A composition or pharmaceutical composition as disclosed is preferably said to be active, functional or therapeutically active or able to treat, prevent and/or delay an infectious disease when it decreases the amount of a *Staphylococcus* species present in a patient or in a cell of said patient or in a cell line or in a cell free in vitro system and preferably means that 99%, 90%, 80%, 70%, 60%, 50%, 40%, 30%, 20%, 10%, 5% or less of the initial amount of a *Staphylococcus* species, is still detectable after treatment. Preferably no *Staphylococcus* species is detectable after treatment. Herein, the expression "amount of *Staphylococcus* species" preferably means alive *Staphylococcus* species. *Staphylococcus* species may be detected using standard techniques known by the artisan such as immunohistochemical techniques using *Staphylococcus* specific antibodies, tube coagulase tests that detect staphylocoagulase or "free coagulase", detection of surface proteins such as clumping factor (slide coagulase test) and/or protein A (commercial latex tests). Alive *Staphylococcus* species may be detected using standard techniques known by the artisan such as microbiological bacterial culture techniques and/or real-time quantitative reverse transcription polymerase chain reaction to assay for bacterial mRNA. Said decrease is preferably assessed in a tissue or in a cell of an individual or a patient by comparison to the amount present in said individual or patient before treatment with the composition or pharmaceutical composition as disclosed herein. Alternatively, the comparison can be made with a tissue or cell of said individual or patient which has not yet been treated with the composition or pharmaceutical composition as disclosed herein in case the treatment is local.

A composition or pharmaceutical composition as disclosed herein may be administered to a subject in need thereof or to a cell, tissue or organ or said patient for at least one day, one week, one month, six months, one year or more.

Accordingly, there is provided a composition or a pharmaceutical composition as disclosed herein, for use as a medicament for the treatment of a subject in need thereof. Preferably, the composition or pharmaceutical composition is use as a medicament in the prevention, delay or treatment of a condition in a subject, wherein the condition is associated with infection with a *Staphylococcus,* such as a coagulase positive- or coagulase-negative *Staphylococcus,* preferably *Staphylococcus aureus* and/or *Staphylococcus epidermidis.*

Further provided is the composition or a pharmaceutical composition as disclosed herein for use as a medicament, wherein the composition or pharmaceutical composition is for systemic or local administration to the subject.

Further provided is the composition or a pharmaceutical composition as disclosed herein for use as a medicament, wherein the condition is selected from the group consisting of bacteraemia, infective endocarditis, prosthetic joint infection, osteomyelitis, indwelling medical device infection and implanted medical device infection.

Further provided is the composition or a pharmaceutical composition as disclosed herein for use as a medicament, wherein the composition or pharmaceutical composition is for systemic or local administration to the subject and wherein the condition is selected from the group consisting of bacteraemia, infective endocarditis, prosthetic joint infection, osteomyelitis, indwelling medical device infection and implanted medical device infection.

Local administration may e.g. be used during surgery, locally at the site of infection or site of implant.

The medical use disclosed herein may be formulated as a product as disclosed herein for use as a medicament for treatment of the stated conditions but can equally be formulated as a method of treatment of the stated conditions using a product as disclosed herein, a product as disclosed herein for use in the preparation of a medicament to treat the stated conditions and use of a product as disclosed herein for the treatment of the stated conditions. Such medical uses are all envisaged by the present invention. The subject in need of treatment, delay and/or prevention of the listed conditions may by any animal subject, preferably a mammal, more preferably cattle, domestic animals like a dog or a cat, or a human subject.

Further provided is the *in vitro* use of an endolysin polypeptide as disclosed herein or a nucleic acid construct as disclosed herein, or an expression construct as disclosed herein, or a host cell as disclosed herein, or a composition or pharmaceutical composition as disclosed herein, as an antimicrobial, preferably as a food additive or as a disinfectant, preferably for coating a medical device. Examples of such use are, but are not limited to, rinsing the cups of a milking device with a composition according to the invention before milking to prevent transmission of *Staphylococci* from cow to cow, cleaning of surfaces in food industry and cleaning chirurgical tools. Such use can be combined with any sterilization method or disinfectant known in the art such as ultrasonic cleaning, irradiation or thermal sterilization, by immersing the equipment in a disinfectant solution such as ethanol, ammonium, iodine and/or aldehyde disinfectant, or by using gas sterilization by retaining the device in a closed atmosphere such as formalin gas or ethylene oxide gas.

Further provided is an *in vitro* method for coating a medical device with an endolysin polypeptide as disclosed herein or a composition or a pharmaceutical composition as disclosed herein, comprising contacting the medical device with an endolysin polypeptide as disclosed herein or a composition or pharmaceutical composition as disclosed herein.

Further provided is the use of a an endolysin polypeptide as disclosed herein or a polynucleotide as disclosed herein, or a nucleic acid construct as disclosed herein, or an expression construct as disclosed herein, or a host cell as disclosed herein, or a composition or pharmaceutical composition as disclosed herein, for detecting a *Staphylococcus,* such as *Staphylococcus aureus* and *Staphylococcus epidermidis,* in an *ex vivo* diagnostic application.

**Table 1: Overview of sequences**

| **SEQ ID NO:** | **Name construct** | **Organism** |
|---|---|---|
| 1 | CHAP-Tw | Bacteriophage Twort |
| 2 | M23-LST | *S. simulans* |
| 3 | SH3b-2638 | Bacteriophage 2638A |
| 4 | CHAP-Tw_SH3b-2638 | Artificial construct |
| 5 | CHAP-Tw_ M23-LST_SH3b-2638 | Artificial construct |
| 6 | M23LST_ CHAP-Tw_SH3b-2638 | Artificial construct |
| 7 | CHAPK | Bacteriophage K |
| 8 | M23-2683 | Bacteriophage 2638A |
| 9 | SH3b -LST | *S. simulans* |
| 10 | SH3b -ALE1 | *S. capitis* |
| 11 | CHAP-SEP | Bacteriophage SEP |
| 12 | CHAP-GH15 | Bacteriophage GH15 |
| 13 | TCHAP-K | Bacteriophage K |
| 14 | M23-ALE1 | *S. capitis* |
| 15 | TCHAP-Tw | Bacteriophage Twort |
| 16 | CHAP-Tw C-terminal linker | Bacteriophage Twort |
| 17 | M23-LST C-terminal linker | *S. simulans* |
| 18 | SH3b-2683 N-terminal linker | Bacteriophage 2638A |
| 19 | CHAP-SEP C-terminal linker | Bacteriophage SEP |
| 20 | CHAP-GH15 C-terminal linker | Bacteriophage GH15 |
| 21 | TCHAP-Tw N-terminal linker | Bacteriophage Twort |
| 22 | M23-ALE1 C-terminal linker | *S. capitis* |
| 23 | SH3b-ALE-1 N-terminal Linker | *S. capitis* |
| 24 | CHAPGH15_M23Ale1_SH3b2638 | Chimeric construct |
| 25 | CHAPGH15_M23Ale1_SH3bAle1 | Chimeric construct |
| 26 | CHAPGH15_M23Ale1_SH3bLST | Chimeric construct |
| 27 | CHAPGH15_M23LST_SH3b2638 | Chimeric construct |
| 28 | CHAPGH15_M23LST_SH3bAle1 | Chimeric construct |
| 29 | CHAPGH15_M23LST_SH3bLST | Chimeric construct |
| 30 | CHAPSEP_M23Ale1_SH3b2638 | Chimeric construct |
| 31 | CHAPSEP_M23Ale1_SH3bAle1 | Chimeric construct |
| 32 | CHAPSEP_M23Ale1_SH3bLST | Chimeric construct |
| 33 | CHAPSEP_M23LST_SH3b2638 | Chimeric construct |
| 34 | CHAPSEP_M23LST_SH3bAle1 | Chimeric construct |
| 35 | CHAPSEP_M23LST_SH3bLST | Chimeric construct |
| 36 | CHAPTw_M23Ale1_SH3b2638 | Chimeric construct |
| 37 | CHAPTw_M23Ale1_SH3bAle1 | Chimeric construct |
| 38 | CHAPTw_M23Ale1_SH3bLST | Chimeric construct |
| 39 | CHAPTw_M23LST_SH3b2638 | Chimeric construct |
| 40 | CHAPTw_M23LST_ SH3bAle1 | Chimeric construct |
| 41 | CHAPTw_M23LST_SH3bLST | Chimeric construct |
| 42 | TCHAPK_M23Ale1_SH3b2638 | Chimeric construct |
| 43 | TCHAPK_M23Ale1_SH3bAle1 | Chimeric construct |
| 44 | TCHAPK_M23Ale1_SH3bLST | Chimeric construct |
| 45 | TCHAPK_M23LST_SH3b2638 | Chimeric construct |
| 46 | TCHAPK_M23LST_SH3bAle1 | Chimeric construct |
| 47 | TCHAPK_M23LST_SH3bLST | Chimeric construct |
| 48 | TCHAPTw_M23Ale1_SH3b2638 | Chimeric construct |
| 49 | TCHAPTw_M23Ale1_SH3bAle1 | Chimeric construct |
| 50 | TCHAPTw_M23Ale1_SH3bLST | Chimeric construct |
| 51 | TCHAPTw_M23LST_SH3b2638 | Chimeric construct |
| 52 | TCHAPTw_M23LST_SH3bAle1 | Chimeric construct |
| 53 | TCHAPTw_M23LST_SH3bLST | Chimeric construct |
| 54 | M23Ale1_CHAPGH15_SH3b2638 | Chimeric construct |
| 55 | M23Ale1_CHAPGH15_SH3bAle1 | Chimeric construct |
| 56 | M23Ale1_CHAPGH15_SH3bLST | Chimeric construct |
| 57 | M23Ale1_CHAPSEP_SH3b2638 | Chimeric construct |
| 58 | M23Ale1_CHAPSEP_SH3bAle1 | Chimeric construct |
| 59 | M23Ale1_CHAPSEP_SH3bLST | Chimeric construct |
| 60 | M23Ale1_CHAPTw_SH3b2638 | Chimeric construct |
| 61 | M23Ale1_CHAPTw_SH3bAle1 | Chimeric construct |
| 62 | M23Ale1_CHAPTw_SH3bLST | Chimeric construct |
| 63 | M23Ale1_TCHAPK_SH3b2638 | Chimeric construct |
| 64 | M23Ale1_TCHAPK_SH3bAle1 | Chimeric construct |
| 65 | M23Ale1_TCHAPK_SH3bLST | Chimeric construct |
| 66 | M23Ale1_TCHAPTw_SH3b2638 | Chimeric construct |
| 67 | M23Ale1_TCHAPTw_SH3bAle1 | Chimeric construct |
| 68 | M23Ale1_TCHAPTw_SH3bLST | Chimeric construct |
| 69 | M23LST_CHAPGH15_SH3b2638 | Chimeric construct |
| 70 | M23LST_CHAPGH15_SH3bAle1 | Chimeric construct |
| 71 | M23LST_CHAPGH15_SH3bLST | Chimeric construct |
| 72 | M23LST_CHAPSEP_SH3b2638 | Chimeric construct |
| 73 | M23LST_CHAPSEP_SH3bAle1 | Chimeric construct |
| 74 | M23LST_CHAPSEP_SH3bLST | Chimeric construct |
| 75 | M23LST_CHAPTw_SH3bAle1 | Chimeric construct |
| 76 | M23LST_CHAPTw_SH3b2638 | Chimeric construct |
| 77 | M23LST_CHAPTw_SH3bLST | Chimeric construct |
| 78 | M23LST_TCHAPK_SH3b2638 | Chimeric construct |
| 79 | M23LST_TCHAPK_SH3bAle1 | Chimeric construct |
| 80 | M23LST_TCHAPK_SH3bLST | Chimeric construct |
| 81 | M23LST_TCHAPTw_SH3b2638 | Chimeric construct |
| 82 | M23LST_TCHAPTw_SH3bAle1 | Chimeric construct |
| 83 | M23LST_TCHAPTw_SH3bLST | Chimeric construct |

### Figure legends

**Figure 1****,** Staphylococcal peptidoglycan and EAD cut sites
   The molecular structure of the *S. aureus* PG repeating unit, which consists of two opposing sugar strands, the stem peptides and the peptide bridge with the cut sited of the EADs, M23 and CHAP, adapted from (adapted from Yikrazuul 2008).
**Figure 2** Turbidity Reduction Assays in human serum and PBS
   Substrate cells of *S*. *aureus* ATCC25904 (A and D), *S*. *epidermidis* BB1336 (B and E) or S. *epidermidis* DSM1798 (C and F) were used to adjust human serum or PBS to an OD of 1. The decrease in OD was measured over 1h for 100, 50, 25, 12.5 and 6.25 nM of LST, M23LST(L)_SH3b2638, CHAPTw(L)_SH3b2638 and CHAPTw_M23LST_SH3b2638. The specific activity was calculated from the steepest slope of a 5-parametic, sigmoidal curve fit. Error bars indicate the Standard Error of the Mean.

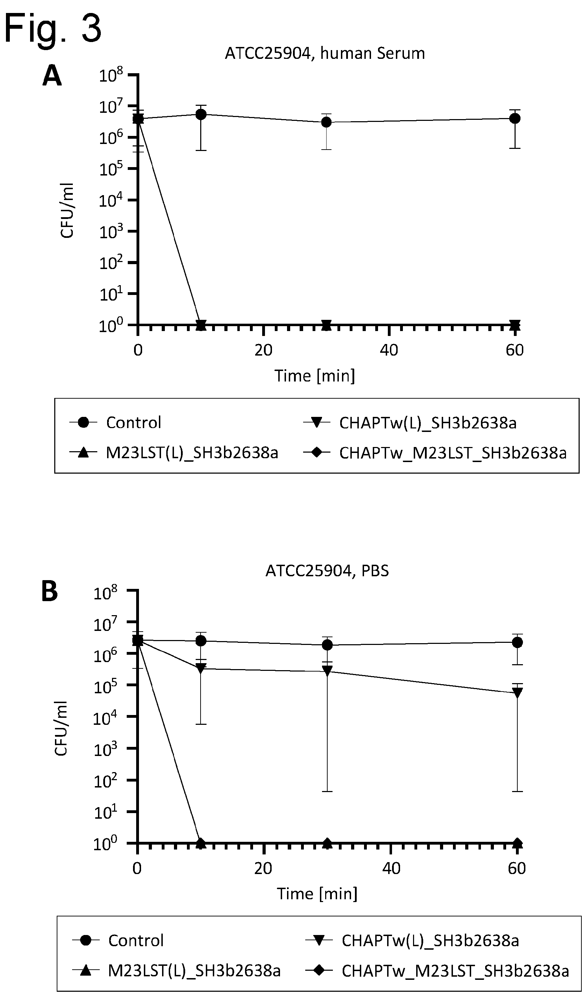

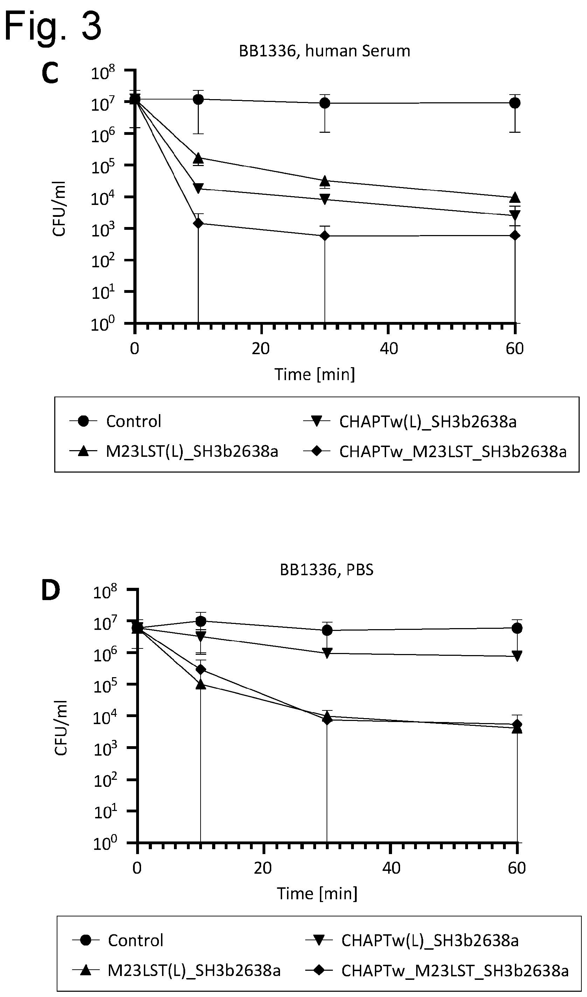

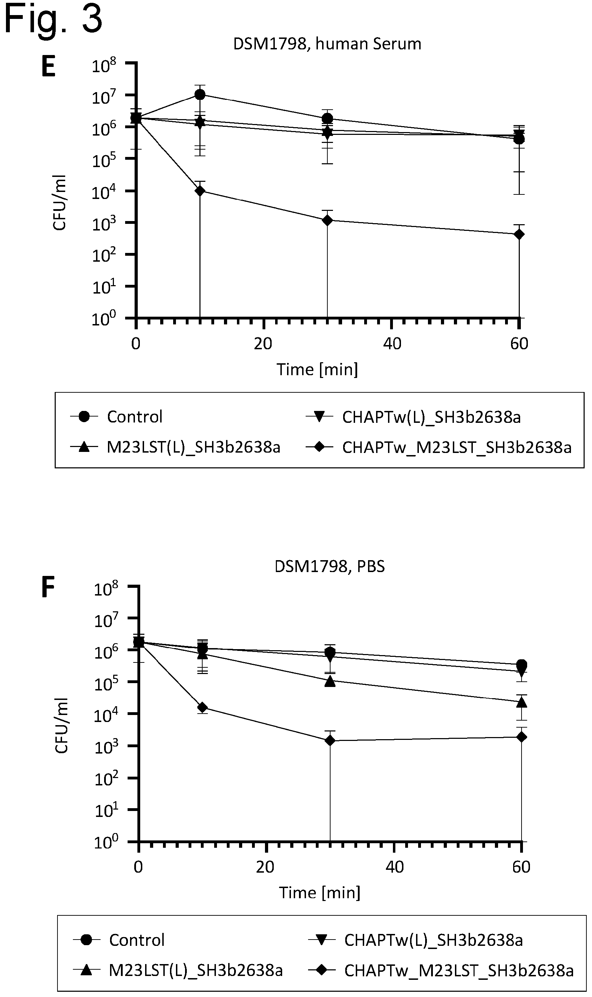

**Figure 3** Time Kill Assays in human serum and PBS
   Buffers (human serum and PBS) were spiked with 10⁶-10⁷ CFU/ml *S*. *aureus* ATCC25904 (A and B), *S. epidermidis* BB1336 (C and D) or *S*. *epidermidis* DSM1798 (E and F). Time kill assays were performed for 1 hour with M23LST(L)_SH3b2638 (triangles up), CHAPTw(L)_SH3b2638 (triangles down) and CHAPTw_M23LST_SH3b2638 (diamonds). Samples were taken, diluted and plated after 0, 10, 30 and 60 minutes, the used PGH concentration was 100 nM, controls without PGHs were included (dots). Error bars indicate the Standard Error of the Mean.

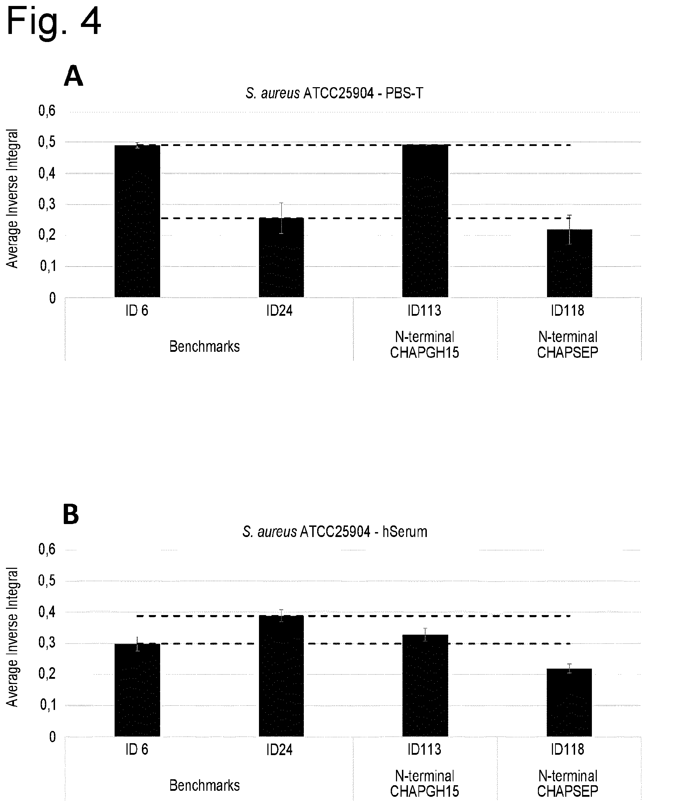

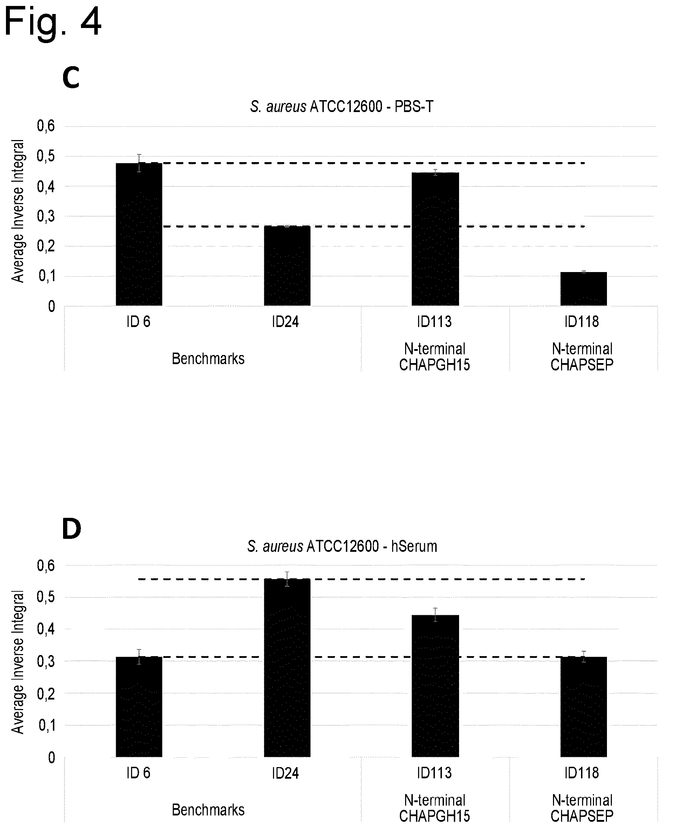

**Figure 4** Turbidity reaction assays *S*. *aureus* in human serum and PBS
   See Table 5 for the genetic make-up of the constructs used.
   A: Turbidity reduction assays (TRA) in PBS-T were performed to evaluate the activity of two endolysins against *S*. *aureus* ATCC25904 in comparison to the activity of two benchmark proteins ID6 (top dashed line) and ID24 (bottom dashed line). The activity of each endolysin was calculated as the mean of the integrals from TRA lysis curves of four endolysin concentrations (40 nM, 20 nM, 10 nM, 5 nM). The average of the inversed means (1-mean) and S.E.M. obtained from biological duplicates is depicted.
   B: Turbidity reduction assays (TRA) in human serum (hSerum) were performed to evaluate the activity of two endolysins against *S*. *aureus* ATCC25904 in comparison to the activity of two benchmark proteins ID6 (bottom dashed line) and ID24 (top dashed line). The activity of each endolysin was calculated as the mean of the integrals from TRA lysis curves of four endolysin concentrations (40 nM, 20 nM, 10 nM, 5 nM). The average of the inversed means (1-mean) and S.E.M. obtained from three biological replicates is depicted.
   C: Turbidity reduction assays (TRA) in PBS-T were performed to evaluate the activity of two endolysins against *S*. *aureus* ATCC12600 in comparison to the activity of two benchmark proteins ID6 (top dashed line) and ID24 (bottom dashed line). The activity of each endolysin was calculated as the mean of the integrals from TRA lysis curves of four endolysin concentrations (40 nM, 20 nM, 10 nM, 5 nM). The average of the inversed means (1-mean) and S.E.M. obtained from biological duplicates is depicted.
   D: Turbidity reduction assays (TRA) in human serum (hSerum) were performed to evaluate the activity of two endolysins against *S*. *aureus* ATCC12600 in comparison to the activity of two benchmark proteins ID6 (bottom dashed line) and ID24 (top dashed line). The activity of each endolysin was calculated as the mean of the integrals from TRA lysis curves of four endolysin concentrations (40 nM, 20 nM, 10 nM, 5 nM). The average of the inversed means (1-mean) and S.E.M. obtained from four biological replicates is depicted.

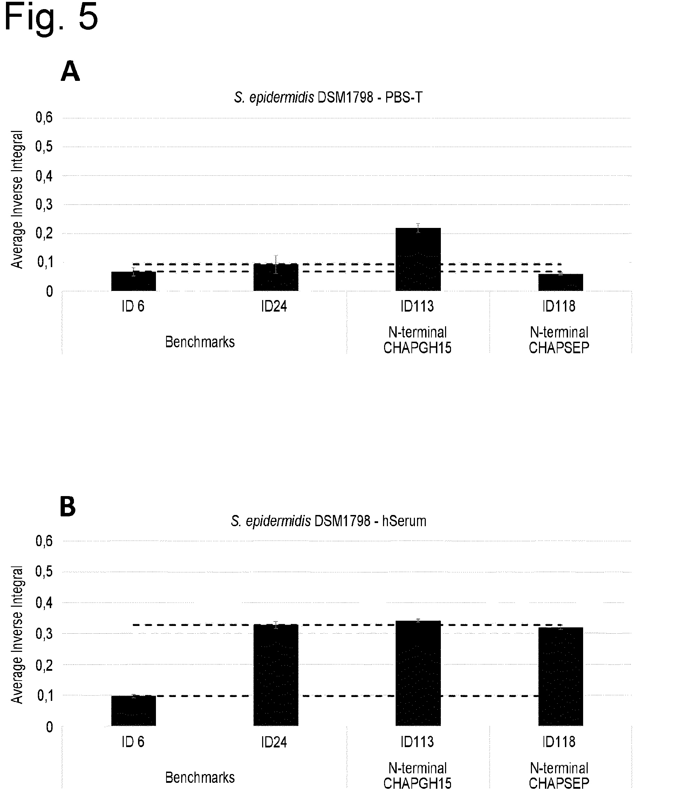

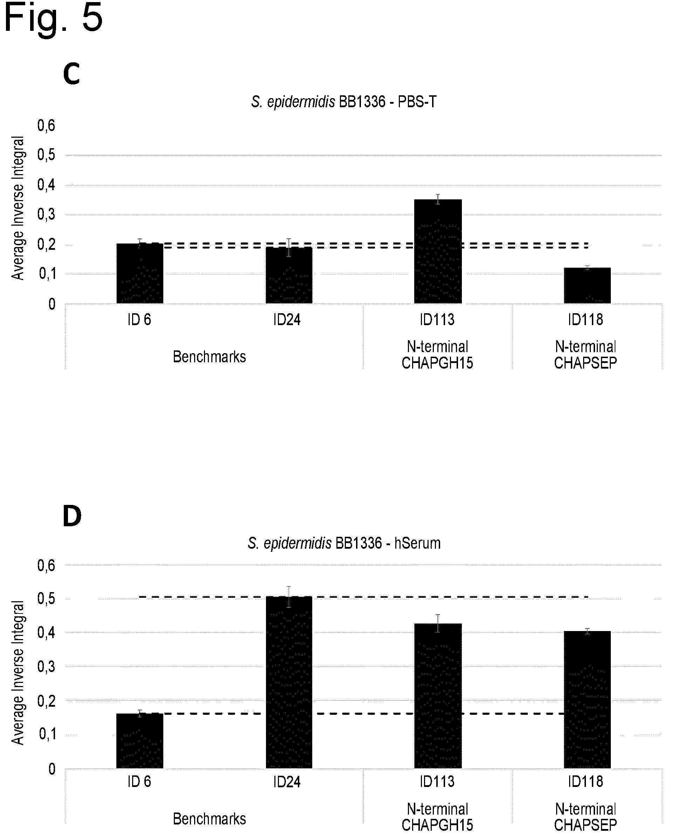

**Figure 5** Turbidity reaction assays *S*. *epidermidis* in human serum and PBS
   See Table 5 for the genetic make-up of the constructs used.
   A: Turbidity reduction assays (TRA) in PBS-T were performed to evaluate the activity of two endolysins against *S*. *epidermidis* DSM1798 in comparison to the activity of two benchmark proteins ID6 (bottom dashed line) and ID24 (top dashed line). The activity of each endolysin was calculated as the mean of the integrals from TRA lysis curves of four endolysin concentrations (80 nM, 40 nM, 20 nM, 10 nM). The average of the inversed means (1-mean) and S.E.M. obtained from biological duplicates is depicted.
   B: Turbidity reduction assays (TRA) in human serum (hSerum) were performed to evaluate the activity of two endolysins against *S*. *epidermidis* DSM1798 in comparison to the activity of two benchmark proteins ID6 (bottom dashed line) and ID24 (top dashed line). The activity of each endolysin was calculated as the mean of the integrals from TRA lysis curves of four endolysin concentrations (80 nM, 40 nM, 20 nM, 10 nM). The average of the inversed means (1-mean) and S.E.M. obtained from three biological replicates is depicted.
   C: Turbidity reduction assays (TRA) in PBS-T were performed to evaluate the activity of two endolysins against *S*. *epidermidis* BB1336 in comparison to the activity of two benchmark proteins ID6 (top dashed line) and ID24 (bottom dashed line). The activity of each endolysin was calculated as the mean of the integrals from TRA lysis curves of four endolysin concentrations (80 nM, 40 nM, 20 nM, 10 nM). The average of the inversed means (1-mean) and S.E.M. obtained from biological duplicates is depicted.
   D: Turbidity reduction assays (TRA) in human serum (hSerum) were performed to evaluate the activity of two endolysins against *S*. *epidermidis* BB1336 in comparison to the activity of two benchmark proteins ID6 (bottom dashed line) and ID24 (top dashed line). The activity of each endolysin was calculated as the mean of the integrals from TRA lysis curves of four endolysin concentrations (80 nM, 40 nM, 20 nM, 10 nM). The average of the inversed means (1-mean) and S.E.M. obtained from three biological replicates is depicted.

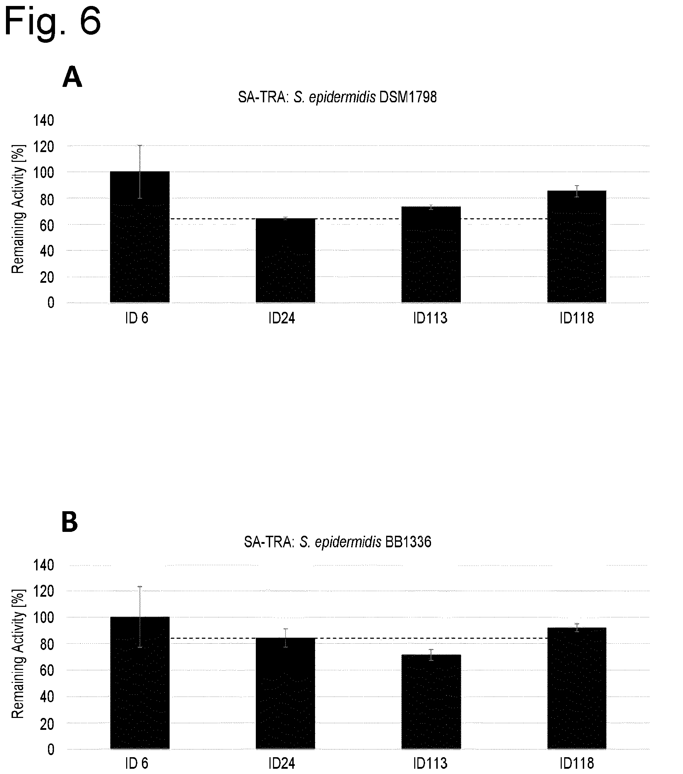

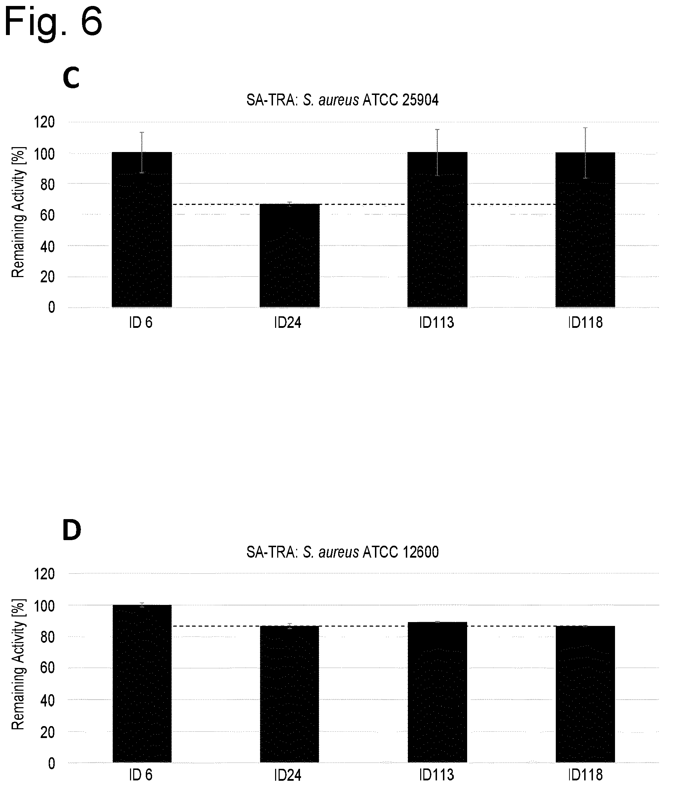

**Figure 6** Stability assays
   A: Turbidity reduction assays in human serum were performed to assess the stability of 4 endolysins on *S. epidermidis* DSM 1798. The stability is expressed as percentage of activity remaining after incubation for 4h at 37 °C compared to activity before incubation. The maximum percentage of remaining activity was set to be 100%. The average and S.E.M based on biological duplicates are presented for the benchmark proteins (ID 6 and ID 24, see Table 5 for their make-up), CHAPGH15_M23LST_SH3bLST (ID 113) and CHAPSEP_M23LST_SH3bLST (ID 118).
   B: Turbidity reduction assays in human serum were performed to assess the stability of 4 endolysins on *S*. *epidermidis* BB1336. The stability is expressed as percentage of activity remaining after incubation for 4h at 37 °C compared to activity before incubation. The maximum percentage of remaining activity was set to be 100%. The average and S.E.M based on biological duplicates are presented for the benchmark proteins (ID 6 and ID 24, see Table 5 for their make-up), CHAPGH15_M23LST_SH3bLST (ID 113) and CHAPSEP_M23LST_SH3bLST (ID 118).
   C: Turbidity reduction assays in human serum were performed to assess the stability of 4 endolysins on *S*. *aureus* ATCC 25904. The stability is expressed as percentage of activity remaining after incubation for 4h at 37 °C compared to activity before incubation. The maximum percentage of remaining activity was set to be 100%. The average and S.E.M based on biological duplicates are presented for the benchmark proteins (ID 6 and ID 24, see Table 5 for their make-up), CHAPGH15_M23LST_SH3bLST (ID 113) and CHAPSEP_M23LST_SH3bLST (ID 118).
   D: Turbidity reduction assays in human serum were performed to assess the stability of 4 endolysins on *S*. *aureus* ATCC 12600. The stability is expressed as percentage of activity remaining after incubation for 4h at 37 °C compared to activity before incubation. The maximum percentage of remaining activity was set to be 100%. The average and S.E.M based on biological duplicates are presented for the benchmark proteins (ID 6 and ID 24, see Table 5 for their make-up), CHAPGH15_M23LST_SH3bLST (ID 113) and CHAPSEP_M23LST_SH3bLST (ID 118).

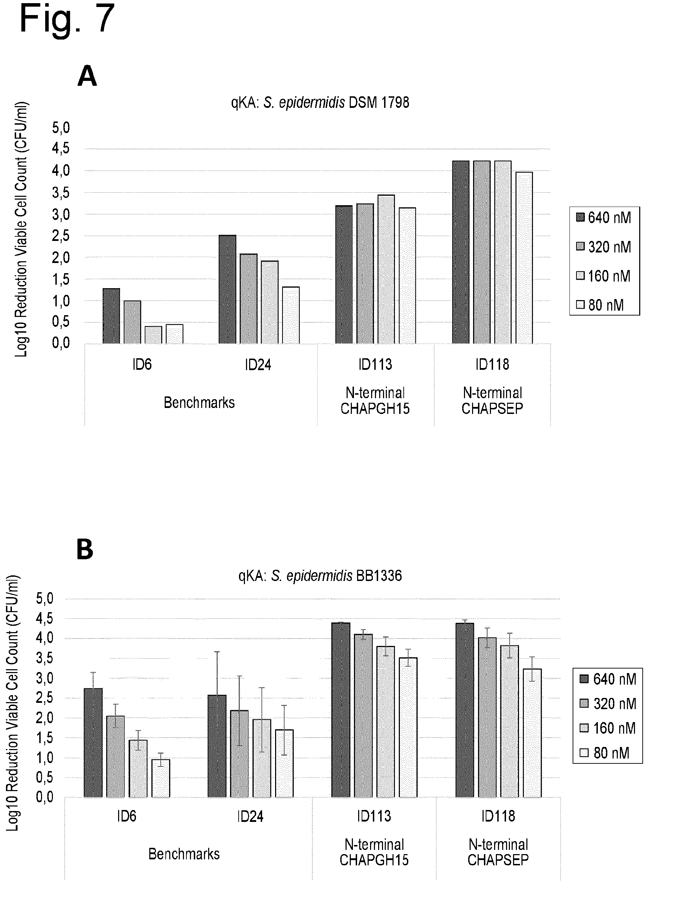

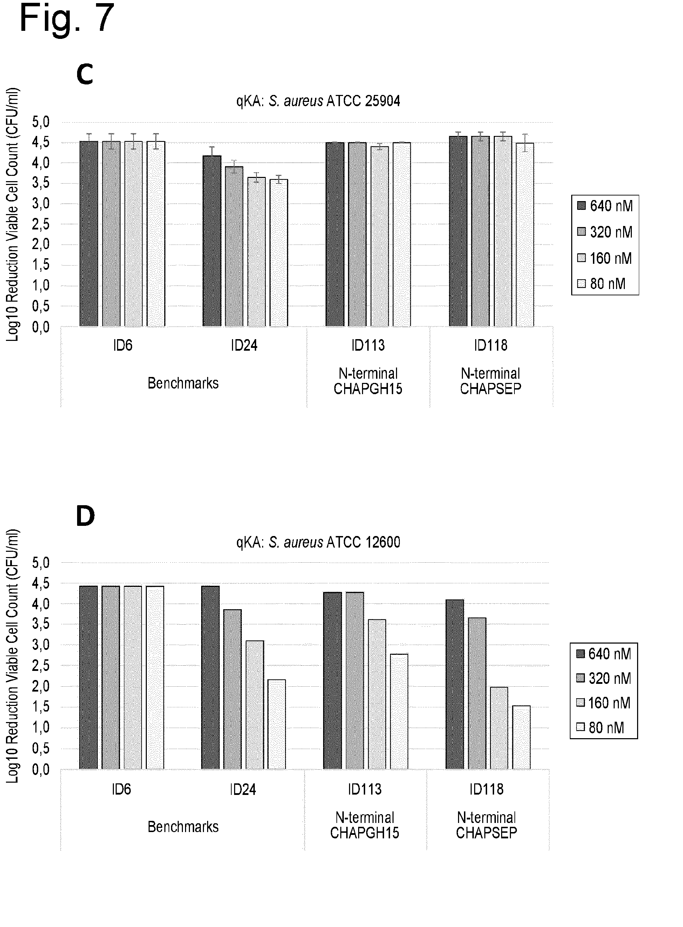

**Figure 7** Quantitative killing assays
   A quantitative killing assay in human serum was performed to assess the log10 reduction in viable cell count [CFU/mL] after exposure to the benchmark proteins (ID6 and ID24, see Table 5 for their make-up) and to enzymes containing a N-terminally located CHAPGH15 or CHAPSEP domain: The decrease in viable cell count of *S*. *epidermidis* DSM 1798 (A), *S*. *epidermidis* BB1336 (B), *S*. *aureus* ATCC 25904 (C), and *S*. *aureus* ATCC 12600 (D) was investigated. The method detection limit values were found to be between 4 and 5 log10 reduction units based on the difference of viable cell count in the negative control and the minimum detectable cell count. The average and S.E.M. based on three biological replicates (B, C) or data based on one assay performance (A, D) are shown.

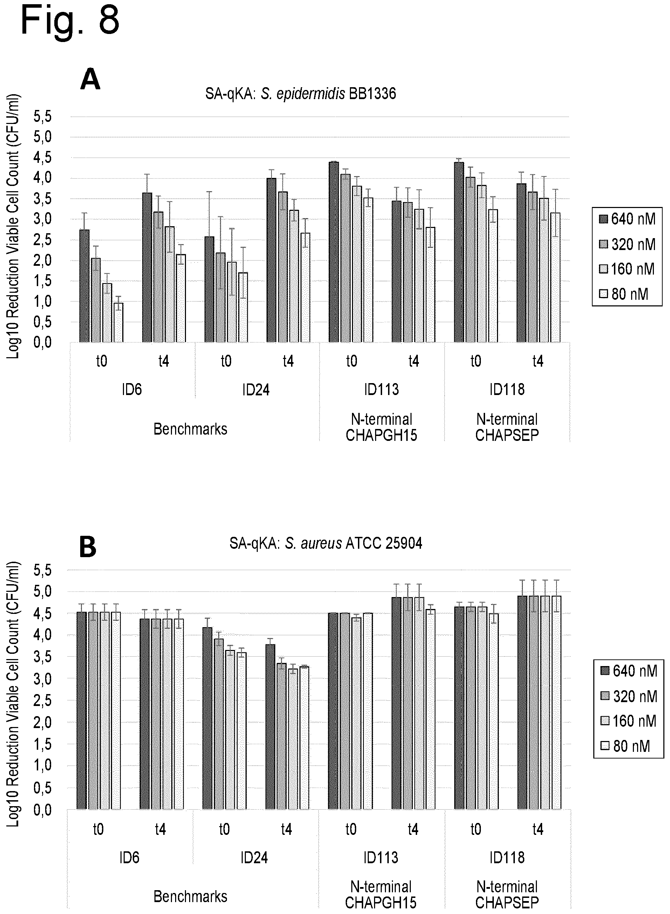

**Figure 8** A stability assay -quantitative killing assay in human serum was performed to assess the stability of the enzymes by comparison of the log10 reduction in viable cell count [CFU/mL] before and after 4 h incubation at 37°C. The exposure to the benchmark proteins (ID6 and ID24) and to enzymes containing a N-terminally located CHAPGH15 or CHAPSEP domain was assessed on *S*. *epidermidis* BB1336 (A) and *S*. *aureus* ATCC 25904 (B). The method detection limit values were found to be between 4 and 5 log10 reduction units based on the difference of viable cell count in the negative control and the minimum detectable cell count. The average and S.E.M. based on three biological replicates (t0) and data based on biological duplicates (t4) are shown.

### Definitions

"Sequence identity" is herein defined as a relationship between two or more amino acid (peptide, polypeptide, or protein) sequences or two or more nucleic acid (nucleotide, polynucleotide) sequences, as determined by comparing the sequences. In the art, "identity" also means the degree of sequence relatedness between amino acid or nucleotide sequences, as the case may be, as determined by the match between strings of such sequences. "Similarity" between two amino acid sequences is determined by comparing the amino acid sequence and its conserved amino acid substitutes of one peptide or polypeptide to the sequence of a second peptide or polypeptide. In a preferred embodiment, identity or similarity is calculated over the whole SEQ ID NO as identified herein. "Identity" and "similarity" can be readily calculated by known methods, including but not limited to those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heine, G., Academic Press, 1987; and Sequence Analysis Primer, Gribskov, M. and Devereux, J., eds., M Stockton Press, New York, 1991; and Carillo, H., and Lipman, D., SIAM J. Applied Math., 48:1073 (1988).

Preferred methods to determine identity are designed to give the largest match between the sequences tested. Methods to determine identity and similarity are codified in publicly available computer programs. Preferred computer program methods to determine identity and similarity between two sequences include e.g. the GCG program package (Devereux, J., et al., Nucleic Acids Research 12 (1): 387 (1984)), BestFit, BLASTP, BLASTN, and FASTA (Altschul, S. F. et al., J. Mol. Biol. 215:403-410 (1990). The BLAST X program is publicly available from NCBI and other sources (BLAST Manual, Altschul, S., et al., NCBI NLM NIH Bethesda, MD 20894; Altschul, S., et al., J. Mol. Biol. 215:403-410 (1990). The well-known Smith Waterman algorithm may also be used to determine identity.

Preferred parameters for polypeptide sequence comparison include the following: Algorithm: Needleman and Wunsch, J. Mol. Biol. 48:443-453 (1970); Comparison matrix: BLOSUM62 from Hentikoff and Hentikoff, Proc. Natl. Acad. Sci. USA. 89:10915-10919 (1992); Gap Penalty: 12; and Gap Length Penalty: 4. A program useful with these parameters is publicly available as the "Ogap" program from Genetics Computer Group, located in Madison, WI. The aforementioned parameters are the default parameters for amino acid comparisons (along with no penalty for end gaps).

Preferred parameters for nucleic acid comparison include the following: Algorithm: Needleman and Wunsch, J. Mol. Biol. 48:443-453 (1970); Comparison matrix: matches=+10, mismatch=0; Gap Penalty: 50; Gap Length Penalty: 3. Available as the Gap program from Genetics Computer Group, located in Madison, Wis. Given above are the default parameters for nucleic acid comparisons.

Optionally, in determining the degree of amino acid similarity, the skilled person may also take into account so-called "conservative" amino acid substitutions, as will be clear to the skilled person. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. Preferred conservative amino acids substitution groups are: valine-leucine-isoleucine, phenylalanine-tyrosine, lysine-arginine, alanine-valine, and asparagine-glutamine. Substitutional variants of the amino acid sequence disclosed herein are those in which at least one residue in the disclosed sequences has been removed and a different residue inserted in its place. Preferably, the amino acid change is conservative. Preferred conservative substitutions for each of the naturally occurring amino acids are as follows: Ala to ser; Arg to lys; Asn to gln or his; Asp to glu; Cys to ser or ala; Gln to asn; Glu to asp; Gly to pro; His to asn or gln; Ile to leu or val; Leu to ile or val; Lys to arg; gln or glu; Met to leu or ile; Phe to met, leu or tyr; Ser to thr; Thr to ser; Trp to tyr; Tyr to trp or phe; and, Val to ile or leu.

A "nucleic acid molecule" or "polynucleotide" (the terms are used interchangeably herein) is represented by a nucleotide sequence. A "polypeptide" is represented by an amino acid sequence. A "nucleic acid construct" is defined as a nucleic acid molecule which is isolated from a naturally occurring gene or which has been modified to contain segments of nucleic acids which are combined or juxtaposed in a manner which would not otherwise exist in nature. A nucleic acid molecule is represented by a nucleotide sequence. Optionally, a nucleotide sequence present in a nucleic acid construct is operably linked to one or more control sequences, which direct the production or expression of said peptide or polypeptide in a cell or in a subject.

"Operably linked" is defined herein as a configuration in which a control sequence is appropriately placed at a position relative to the nucleotide sequence coding for the polypeptide of the invention such that the control sequence directs the production/expression of the peptide or polypeptide of the invention in a cell and/or in a subject. "Operably linked" may also be used for defining a configuration in which a sequence is appropriately placed at a position relative to another sequence coding for a functional domain such that a chimeric polypeptide is encoded in a cell and/or in a subject.

"Expression" is construed as to include any step involved in the production of the peptide or polypeptide including, but not limited to, transcription, post-transcriptional modification, translation, post-translational modification and secretion.

A "control sequence" is defined herein to include all components which are necessary or advantageous for the expression of a polypeptide. At a minimum, the control sequences include a promoter and transcriptional and translational stop signals. Optionally, a promoter represented by a nucleotide sequence present in a nucleic acid construct is operably linked to another nucleotide sequence encoding a peptide or polypeptide as identified herein.

The term "transformation" refers to a permanent or transient genetic change induced in a cell following the incorporation of new DNA (i.e. DNA exogenous to the cell). When the cell is a bacterial cell, as is intended in the present invention, the term usually refers to an extrachromosomal, self-replicating vector which harbors a selectable antibiotic resistance.

An "expression vector" may be any vector which can be conveniently subjected to recombinant DNA procedures and can bring about the expression of a nucleotide sequence encoding a polypeptide of the invention in a cell and/or in a subject. As used herein, the term "promoter" refers to a nucleic acid fragment that functions to control the transcription of one or more genes or nucleic acids, located upstream with respect to the direction of transcription of the transcription initiation site of the gene. It is related to the binding site identified by the presence of a binding site for DNA-dependent RNA polymerase, transcription initiation sites, and any other DNA sequences, including, but not limited to, transcription factor binding sites, repressor and activator protein binding sites, and any other sequences of nucleotides known to one skilled in the art to act directly or indirectly to regulate the amount of transcription from the promoter. Within the context of the invention, a promoter preferably ends at nucleotide -1 of the transcription start site (TSS).

A "polypeptide" as used herein refers to any peptide, oligopeptide, polypeptide, gene product, expression product, or protein. A polypeptide is comprised of consecutive amino acids. The term "polypeptide" encompasses naturally occurring or synthetic molecules.

The sequence information as provided herein should not be so narrowly construed as to require inclusion of erroneously identified bases. The skilled person is capable of identifying such erroneously identified bases and knows how to correct for such errors.

In this document and in its claims, the verb "to comprise" and its conjugations is used in its nonlimiting sense to mean that items following the word are included, but items not specifically mentioned are not excluded. In addition the verb "to consist" may be replaced by "to consist essentially of" meaning that a product or a composition or a nucleic acid molecule or a peptide or polypeptide of a nucleic acid construct or vector or cell as defined herein may comprise additional component(s) than the ones specifically identified; said additional component(s) not altering the unique characteristic of the invention. In addition, reference to an element by the indefinite article "a" or "an" does not exclude the possibility that more than one of the elements is present, unless the context clearly requires that there be one and only one of the elements. The indefinite article "a" or "an" thus usually means "at least one". The word "about" or "approximately" when used in association with a numerical value (e.g. about 10) preferably means that the value may be the given value (of 10) more or less 10% of the value.

### Examples

**Example 1:** Differences in lytic activity of enzymes with either a single EAD (CHAP or M23) or a combination of both.

### Introduction

The Gram-positive bacteria of the genus *Staphylococci* are a major concern in human health care, mostly due to the increasing prevalence of antibiotic-resistance. They can infect virtually any tissue, including the skin, soft tissues, bones, the heart, the lung and even the brain (Tong, 2015). Additionally, biofilms can serve as centres of infections for systemic infections and sepsis, for example on prosthetic devices and catheters. Among the most prevalent bacterial species are the coagulase-positive *S*. *aureus* and the coagulase-negative *S. epidermidis.* Infections with *Staphylococci* are often difficult to treat due to high levels of antibiotics resistance and the physically restricted access to the centres of infection. There is a high demand for novel drugs, active against *Staphylococci.*

Peptidoglycan hydrolases (PGHs) can cleave specific bonds within the peptidoglycan (PG) network of bacteria and have been shown to be active against biofilms. Their high lytic activity makes PGHs potent anti-staphylococcal agents. Endolysins are highly specific, phage-derived PGHs, active against both drug-sensitive and resistant bacteria (Schmelcher et al. 2012). As potential alternatives to antibiotics, they have undergone investigations *in vitro, in vivo* and are under trial in several clinical studies (Kashani et al. 2017). PGHs of *Staphylococci* regularly display a domain-like architecture, consisting of enzymatically active domains (EADs) and cell-wall-binding domains (CBDs). The high specificity of staphylococcal PGHs may be attributed to their CBDs, which regularly feature an SH3b-fold. The structures of staphylococcal endolysin SH3b domains have been solved and display great homology to the bacteriocins lysostaphin (LST) and ALE1, suggesting a common recognition site in the PG.

The EADs are more divers and can be grouped according to their structure and cleavage site within the PG. We investigated two EAD structures, the cysteine, histidine dependent amidohydrolase/peptidase (CHAP) and the M23 endopeptidase domain. CHAP domains are frequently found in staphylolytic endolysins, for example in phage Twort or phage K (Korndörfer et al. 2006). Depending on the CHAP domain present, cleavage can occur at different locations in the PG, including the amide bond of the sugar backbone to the stem peptide and the link of the stem peptide to the peptide cross bridge (Figure 1). M23 domains, have only been found in one endolysin (phage 2638), but are also present in the staphylococcal bacteriocin LST and its homologue ALE1. The M23 domains of LST and ALE1 cleave the pentaglycine cross-bridge, which connects adjacent stem peptides in the PG of *S*. *aureus,* whereas the M23 domain of phage 2638 cuts between the peptide bridge and the stem peptide (Gründling et al. 2006; Schmelcher et al. 2015 JAC).

Previously, effectivity was positively assessed in milk for combinations of separate PGHs against *S. aureus* with orthogonal cut sites in the PG (Verbree et al. 2018). This may be because cleavage of one bond within the PG network may result in better accessibility of different bonds within the three-dimensional PG network.

Herein, we investigated the combination of the M23 endopeptidase from LST (M23LST) and the CHAP domain from the endolysin of phage Twort (CHAPTw) on a single chimeric molecule, specifically the effectivity on *S*. *epiderimidis* and *S. aureus* was assessed.

### Materials and Methods

To quantify the differences in activity between different PGH constructs we compared up to four enzymes in quantitative assays, namely Turbidity Redcution Assays (TRAs) and Time Kill Assays (TKAs) using PBS and human serum.

The enzymes tested, were recombinantly produced in *E*. *coli* BL21-GOLD (DE3) and are indicated in Table 2. Their purification was performed by Cation Exchange Chromatography using a HiTrap SP-FF cation exchange columns on an ÄKTA FPLC device (GE Healthcare, Uppsala, Sweden). The chosen constructs contained either the M23LST, the CHAPTw domain, or both. Three of the four enzymes contained the CBD of phage 2638 (SH3b2638). The fourth enzyme was LST, which contains the native SH3b domain of LST and was only used in TRAs as a control. M23(L)_SH3b2638, CHAPTw(L)_SH3b2638 and LST were reconstituted from lyophilized samples, CHAPTw_M23LST_SH3b2638 was thawed on ice from a frozen aliquot. Consequently, all enzymes had comparable concentrations and were stored at 4°C on ice, for the duration of the experimental procedures. The "(L)" designation in the protein name indicates the part of which domain was used to construct the linker sequence between EAD and SH3b.

**Table 2, Peptidoglycan Hydrolases used in this study**

| **Protein** | **Concentration [mg/ml]** | **Molecular weight [g/mol]** |
|---|---|---|
| M23(L)_SH3b2638 | 0.85 | 27546 |
| CHAPTw(L)_SH3b2638 | 1.02 | 30875 |
| CHAPTw_M23LST_SH3b2638 | 1.17 | 46722 |
| LST | 1.45 | 27075 |

Enzymatic activities were measured on coagulase-positive and -negative *Staphylococci,* using three well-characterized staphylococcal strains listed in Table 3. Appropriate safety measures were taken for work with Biosafety Level 2 organisms throughout all experiments. All strains were named according to their source and were grown in test tubes in tryptic soy broth (TSB), shaking at 150 rpm, at 37°C. TRAs and TKAs were performed in PBS and additionally in human serum, to better mimic a systemic infection scenario and elucidate potential differences in activity. All other buffers and media used in this study are indicated in Table 4.

**Table 3, Staphylococcal strains used in this study**

| **Species** | **Strain** | **Source** | **Properties** |
|---|---|---|---|
| *S. epidermidis* | (Winslow and Winslow) Evans 1916 | DSMZ 1798 | FDA strain PCI 1200 |
| *S. epidermidis* | BB1336 | BB1336 | Clinical isolate of Methicillin Resistant S. *epidermidis* |
| *S. aureus* | Newman | ATCC 25904 | Clinical isolate (osteomyelitis) |

TRAs were performed essentially as previously described (Schmelcher et al. 2015). In brief, substrate cells of strains ATCC25904, BB1336, DSM1798 were grown in TSB at 37 °C until mid-log phase (OD₆₀₀ of 0.5 - 0.6), harvested at 7000 g in a Beckman Coulter JA-10 rotor and washed two times with 1/10 of the initial culture volume substrate cell buffer. The cells were frozen at - 80 °C in 200 µl aliquots. A 2-fold dilution series of each PGH ranging from 200 nM to 12.5 nM was prepared in the two different media (PBS, human serum). Bacterial suspensions were prepared from frozen substrate cells in each buffer, and 100 µl of the bacterial suspensions were mixed with 100 µl of each dilution of the PGHs in a 96-well plate, so that the initial OD₆₀₀ of the suspension was 1. The decrease in optical density over time was monitored for 1 h at 30 s intervals with a Fluostar Omega plate reader (BMG Labtech, Ortenberg, Germany). The resulting lysis curves were corrected for the negative controls (no enzyme) and the steepest slope was determined after fitting the data points to a 5-parametric sigmoidal function using SigmaPlot 13 (Systat Software, San Jose), as described before (Korndörfer 2006). The specific activity of each PGH, expressed as ΔOD₆₀₀ min⁻¹ µM⁻¹, was determined within the linear activity range of the enzymes.

TKAs were performed essentially as previously described (Verbree et al. 2018). The two different media (PBS, human serum) were spiked with approximately 10⁶ CFU/ml. Equimolar amounts of PGHs were mixed with the spiked buffers, resulting in final PGH concentrations of 100 nM, and the mixtures were stored at 37°C without agitation. After 0, 10, 30 and 60 minutes, samples were taken, diluted in PBS, plated on TSA plates and incubated overnight at 37 °C. Bacterial numbers were determined the following day. A negative control (no enzyme) was included for all time points.

**Table 4, Buffers, Media and Chemicals used in this study**

| **Buffer/Substance** | **Ingredient** | **Manufacturer** | **Article** | **Lot** | **Amo unt** |
|---|---|---|---|---|---|
| Tryptic Soy Broth (TSB) | Tryptic Soy Broth Powder | BioLife | 4012302 | LF0103 | 30 g/L |
| TSB Agar (TSA) | Tryptic Soy Broth Powder | BioLife | 4012302 | LF0103 | 30 g/L |
| | Agar-Agar, Kobe I | Roth | 5210,5 | 388272 789 | 1.4 g/L |
| Phosphate buffered Saline (PBS) | PBS | ThermoFisher Scientific | 14190169 | | - |
| Substrate cell buffer | NaCl | Sigma-Aldrich | 71380-1KG | BCCB8 985 | 8.77 g/L |
| | Na₂HPO_{4 . 12H20} | Sigma-Aldrich | 71650-1KG | BCBZ8 245 | 3.58 g/L |
| adjust pH to 7.5 | Glycerol | Roth | 3783,2 | 472541 66 | 250 ml |
| Human serum | Human serum from human male AB plasma, USA origin, sterile filtered | Sigma-Aldrich | H4522-100ML | | - |
| H₂O | PureLab Chorus 1 | ELGA Labwater | Serial-Nr.: CRS00004702 | | |
| pH was adjusted with | HCl | VWR Chemicals | 20252,29 | 19H224009 | |
| | NaOH | Sigma-Aldrich | 71690-1KG | BCBZ3 631 | |

### Results and discussion

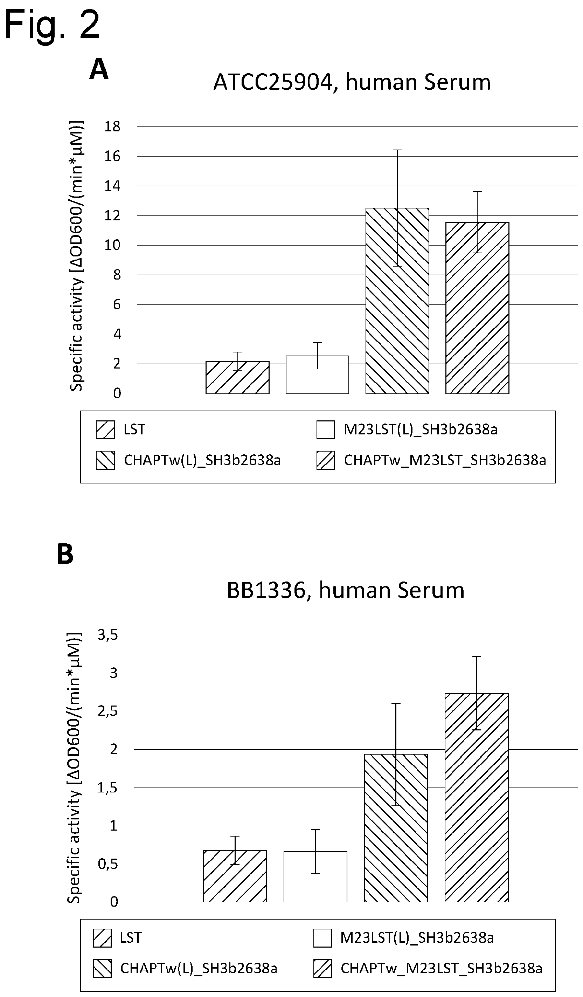

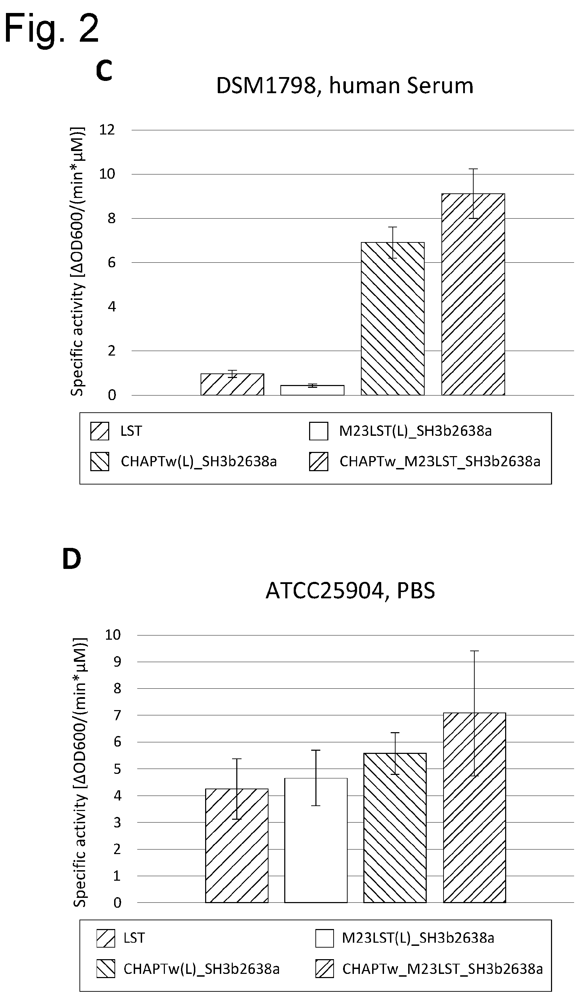

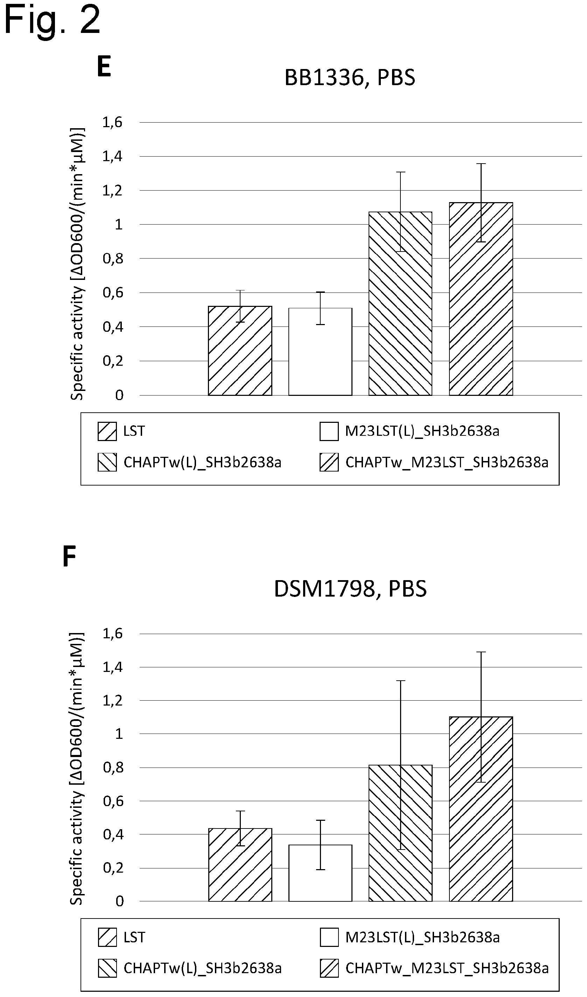

The goal of this project was to explore the differences in lytic activity of enzymes with either a single EAD (CHAP or M23) or a combination of both, against different representatives of the *Staphylococci.* To closely mimic a systemic infection scenario, we performed our analysis in human serum, in addition to PBS. We also aimed to demonstrate the synergy between M23 and CHAP domains within the same molecule. Towards this end, we performed TRAs and TKAs for three different enzymes, namely M23LST(L)_SH3b2638, CHAPTw(L)_SH3b2638 and CHAPTw_M23LST_SH3b2638 in human serum and PBS. Interestingly, the performance of CHAPTw_M23LST_SH3b2638 was superior or at least equal to that of the other enzymes tested. In TRAs, the specific activity of enzymes is measured and expressed as their ability to reduce the optical density of a bacterial suspension. In all assays those constructs harbouring a CHAP domain performed significantly better than those constructs with an M23 domain only, namely LST and M23LST(L)_SH3b2638 (Figure 2). This was particularly true for the coagulase-negative *S*. *epidermidis* strains BB1336 and DSM1798, where very low enzymatic activities were measured (Figure 2B, C, E, F). This may be explained by their varying peptidoglycan structure, which does not display the high abundance of pentaglycine bridges connecting adjacent stem peptides, observed in *S. aureus.* For all constructs containing a CHAP domain, the specific activity was increased by the presence of human serum (Figure 2A, B, C), compared to the activity in PBS (Figure 2D, E, F). This could be of advantage, considering a large number of possible centres of infections can only be reached by systemic administration of PGHs.

In addition, in five of the six tested settings, CHAPTw_M23LST_SH3b2638, which harbors both EADs had the highest specific activity of all constructs. This superiority may be attributed to the synergistic action of both EADs. To further investigate the benefit of an M23 and a CHAP domain within one molecule, we next performed TKAs.

In TKAs the reduction of a defined number of bacteria is measured over a certain amount of time, in our case 10, 30 and 60 minutes. We performed the assay in human serum and in PBS spiked with the three previously mentioned bacterial strains and the enzymes M23(L)_SH3b2638, CHAPTw(L)_SH3b2638 and CHAPTw_M23LST_SH3b2638.

Overall, CHAPTw_M23LST_SH3b2638 performed better or equal to all other enzymes, illustrating the advantage of combining the two different EADs on the same molecule (Figure 3). Particularly, the for the two coagulase-negative strains the advantage of combining the two EADs was apparent. While we observed strong killing in human serum with all enzymes for *S*. *aureus,* the differences between enzymes were more pronounced for *S*. *epidermidis* (Figure 3A, C, E). CHAPTw_M23LST_SH3b2638 reduced the bacterial numbers of BB133 by 4 log units already after 10 minutes, which was approximately one and two log units better than M23(L)_SH3b2638 and CHAPTw(L)_SH3b2638, respectively (Figure 3). For DSM1798, the CFU counts of the latter two enzymes hardly deviated from the control at all, whereas CHAPTw_M23LST_SH3b2638 reached reduction of 3 log units within 60 minutes (Figure 3E).

In PBS, CHAPTw(L)_SH3b2638 displayed very little activity against all strains, as CFU counts remained close to the control (Figure 3B, D, F). As CHAPTw(L)_SH3b2638 was active in human serum, the conditions found in PBS, such as ion or protein concentrations, may have been suboptimal for its activity. As observed in human serum, a faster and better reduction in CFU counts was observed for CHAPTw_M23LST_SH3b2638 for strain DSM1798 (Figure 3 F). Interestingly the susceptibility of DSM1798 to M23LST(L)_SH3b2638 was very low in both, human serum and PBS compared to CHAPTw_M23LST_SH3b2638.

### Example 2: Differences in lytic activity of enzymes with either a single EAD (CHAP or M23) or a combination of both.

In example 2, we investigated the combination of the M23 endopeptidase from LST (M23LST) and two different CHAP domains as in example 1 on a single chimeric molecule; specifically the effectivity on *S. epiderimidis* and *S*. *aureus* was assessed. A listing of the constructs used in this example is depicted in Table 5 here below. The "(L)" designation in the protein name indicates the part of which domain was used to construct the linker sequence between EAD and SH3b.

**Table 5**

| **ID of construct** | **Construct make-up** |
|---|---|
| 6 | M23-LST(L)_SH3b2638 |
| 24 | CHAPTw(L)_SH3b2638 |
| 113 | CHAPGH15_M23LST_SH3bLST |
| 118 | CHAPSEP_M23LST_SH3bLST |

### Materials and methods

### Turbidity Reduction Assay

Turbidity reduction assays (TRA) were performed to assess the activity of endolysins in PBS-T or human serum (hSerum) against the staphylococcal strains *S*. *aureus* ATCC25904, *S. aureus* ATCC12600, methicillin-susceptible *S*. *epidermidis* DSM1798 (MSSE) and methicillin-resistant *S*. *epidermidis* BB1336 (MRSE).

For the generation of substrate cells of *S*. *epidermidis* DSM 1798, *S. epidermidis* BB1336, *S*. *aureus* ATCC 25904 and *S. aureus* ATCC 12600, 5 ml Tryptic Soy Broth (TSB) per 500 ml culture volume (CV) were inoculated from a cell bank aliquot of the desired strain and incubated overnight (O/N) at 37 °C and 180 rpm (Kühner, ISF1-X), which were used as default settings if not mentioned otherwise. Sterile working conditions were applied to keep the substrate cells free of contaminations. 5 ml O/N culture was used per 500 ml CV to inoculate the TSB medium (Tryptic Soy Broth, Applichem) and the culture was incubated until reaching an optical density at 600 nm (OD₆₀₀ₙₘ) of 0.5-0.6 (Thermo Fisher Scientific, Nanodrop One^{C}). The culture was transferred on ice and kept cooled during the subsequent procedure. Cells were harvested by centrifugation at 5'000 x g at 4 °C for 30 min (Hermle, ZK 496). The resulting pellet was resuspended in 1/10 cold *S. aureus* buffer (10 mM Na2HPO4, 150 mM NaCl, 25% glycerol, Ph 7.5) of the initial CV. Washed cells were pelleted again by centrifugation at 5'000 x g at 4 °C for 15 min (Hermle, Z 446 K) and resuspended in 1/100 cold *S. aureus* buffer of the initial CV. 300 µl and 150 µl aliquots were stored at -80 °C (Thermo Fisher Scientific, ULT2090-10-V) until use.

To perform a TRA either PBS-T (PBS with 0.01% Tween-20) or hSerum (Sigma-Aldrich) was used. Aliquots of hSerum were made by thawing 1 L of serum at RT on a microplate shaker at 150 rpm (Fisher Scientific, 88861024) initially until almost all serum was melted and was then transferred to 4 °C in the fridge to further thaw O/N. Flakes appearing in the serum were pelleted by centrifugation at 5'300xg for 10 min at 4°C, supernatant was aliquoted and hSerum aliquots were frozen at -20 °C. Prior to a TRA, a hSerum aliquot was thawed at 30°C in a water bath, sterile filtered (0.45 µm) and stored on ice until use. The endolysins were stored at -80 °C, a 100 µl aliquot was thawed on ice to test in an assay but was discarded and not refrozen after use to avoid repeated freeze-thaw cycles. If the 100 µl aliquots were used up, a 1 ml aliquot was thawed, aliquoted in 100 µl PCR strips and was frozen at -80 °C. In the bottom row of a 96-well F-bottom plate provided with ~200 µl hSerum or PBS-T, a pre-dilution of equimolar concentration (160 nM for *S*. *aureus* and 320 nM for *S*. *epidermidis* strains) for each endolysin tested was prepared. Subsequently, a two-fold dilution series was made in 100 µl provided hSerum or PBS-T by mixing four times and discarding the last 100 µl. Resulting bubbles were bursted with sterile toothpicks or single-use needles to avoid any interference with the measurements. Prior to the assay, the frozen but viable substrate cells were kept on dry ice, were thawed and resuspended in hSerum or PBS-T. The substate cell suspension was adjusted to reach an OD₆₀₀ₙₘ of 2.0 by measuring the OD of a 1:1 mixture in hSerum or PBS-T in semi-micro cuvettes resulting in an OD₆₀₀ₙₘ of 1.0 ±5% (Thermo Fisher Scientific, Nanodrop One^{C}). From the adjusted cell suspension, 100 µl was quickly added with multichannel pipettes per well by reverse pipetting (including a substrate only cell negative control and a buffer only blank) resulting in a final volume of 200 µl and enzyme concentrations of 40 nM, 20 nM, 10 nM, 5 nM tested against *S*. *aureus* and 80 nM, 40 nM, 20 nM, 10 nM against *S*. *epidermidis* substrate cells. The measurement was started immediately and the decrease in optical density at 600 nm was measured in intervals of 10 seconds for 151 cycles (~25 min) at 30 °C with pulsed shaking of 5 seconds. The resulting data was extracted, the blank was subtracted, normalized and control corrected values were used to calculate for each tested concentration the integrals of the lysis curves measured for 25 min. To evaluate the activity of each endolysin, the mean of the integrals from the four tested concentrations was determined and the average was inversed (1-mean). The inverse averaged integrals of each endolysin from two, three or four performed biological replicates (depending on enzyme selection process, strain and buffer) were averaged and the standard error of the mean (S.E.M) was calculated.

### Stability Assay - TRA

The stability of endolysins was determined by turbidity reduction assays (TRA) in human serum before (t=0) and after incubation for 4h at 37 °C (t=4h). Substrate cells of *S*. *epidermidis* DSM 1798, *S. epidermidis* BB1336, *S. aureus* ATCC 25904 and *S*. *aureus* ATCC 12600, were prepared as described above. Human serum was thawed at 30°C in a water bath, sterile filtered (0.45 µm) and stored on ice until use. For the measurements at t=0, a pre-dilution of equimolar concentration (160 nM for *S*. *aureus* and 320 nM for *S*. *epidermidis* strains) for each endolysin tested was prepared in a 96-multiwell plate provided with 200 µl human serum. Thereof, a two-fold dilution series was performed in 100 µl human serum. The frozen but viable substrate cells were resuspended in human serum, adjusted to an OD₆₀₀ₙₘ of 2.0 and the bacterial suspension was mixed at a 1:1 ratio with the diluted enzymes, resulting in an OD₆₀₀ₙₘ of 1.0. Final enzyme concentrations of 40, 20, 10 and 5 nM were tested on *S. aureus* strains while 80, 40, 20 and 10 nM were applied on *S*. *epidermidis* strains. For the measurements at t=4h, a pre-dilution of 0.1 mg/mL in human serum was prepared in microcentrifuge tubes and incubated for 4h at 37 °C. The samples were then prepared in the same manner as for measurements at t=0. Human serum was used for the blank measurement and a 1:1 mixture of bacterial cells with human serum as negative control. Reductions in turbidity due to cell lysis were monitored in time intervals of 10 seconds for 151 cycles at 30 °C by measuring the optical density. The inverse integral of the curves representing the optical density changes was determined and the average of all four concentrations per enzyme calculated. Stability was expressed as a percentage of activity remaining after incubation (t=4h) compared to activity before incubation (t=0).

### Quantitative Killing Assay

Cultures of *S*. *epidermidis* DSM 1798, *S*. *epidermidis* BB1336, *S. aureus* ATCC 25904, and *S*. *aureus* ATCC 12600 in TS broth were incubated overnight at 37°C and 180 rpm. The precultures were diluted 1:25 in 10 ml TS broth and incubated at 37°C and 180 rpm to mid log phase OD₆₀₀~0.5-0.6. The OD₆₀₀ was adjusted to 0.5 and the cultures were stored on ice. Human serum was thawed at 30°C and sterile filtered (0.45 µm) before storage on ice. Endolysins of 640,320,160, and 80 nM in human serum were tested on *S. epidermidis* and endolysins of 160, 80, 40, and 20 nM in human serum were tested on *S. aureus* in a 96-multiwell plate. The bacterial cells were diluted 1:10 in human serum and 100 µl thereof were mixed with 100 µl of double concentrated protein solution per well. Human serum mixed with the bacterial cells served as control. The samples were incubated for 30 min at 37°C and 180 rpm. To inhibit enzymatic activity after the incubation, 20 µl of 10-fold concentrated stopping buffer (Trisodium citrate dehydrate, 11.4g/L; Citric acid (monohydrate), 13.6g/L) were added per well. Serial dilutions (1:10, 1:100, 1:1000). were prepared in stopping buffer, Thereof, 5.5 µl were spotted on a square LB agar plate for the determination of viable cell count after overnight incubation at 37°C. Based on the method, the limit of detectable cell count was 200 CFU/ml. The limit of detection was calculated by subtracting the log-transformed value of 200 from the log-transformed value of the cellular concentration of the control. For the calculation of the log10 reduction in viable cell counts, the log-transformed value in viable cell count was subtracted from the log10 of the viable cell count of the control. The average and S.E.M. were calculated based on the obtained values of log10 reduction in viable cell count.

### Stability Assay - qKA

Protein solutions of 1280 nM were prepared in human serum in microcentrifuge tubes. Their activity against *S. epidermidis* BB1336 and S. *aureus* ATCC 25904 was assessed according to the protocol of the quantitative killing assay before (t=0) and after (t=4h) the incubation of the protein solutions for 4 h at 37°C.

### Comparison of CHAPGH15_M23LST_SH3bLST and CHAPSEP_M23LST_SH3bLST (double EAD constructs)to M23-LST(L)_SH3b2638 and CHAPTw(L)_SH3b2638 (single EAD benchmark constructs)

The activities and stabilities of the constructs as determined by TRAs, SA-Ts, qKAs and SA-Qs were compared to those of the two single EAD constructs M23-LST(L)_SH3b2638 and CHAPTw(L)_SH3b2638. A construct was defined to have better TRA activity if the lower S.E.M. boundary of the inverse integral of the construct exceeded the average inverse integral value of either one of the single EAD constructs. For SA-Ts, a construct was defined to have better stability if the lower S.E.M. boundary of the remaining activity of the construct exceeded the average of the remaining activity of either one of the single EAD constructs. For qKAs, a construct was defined to have better activity if the lower S.E.M. boundary of the log reduction of the construct exceeded the average log reduction of either one of the single EAD constructs. For SA-Qs the same was defined for the log reduction of all constructs after the 4h incubation in human serum at 37°C.

### Results and discussion

We compared the two constructs with two EADs with the constructs with a single EAD in two orthogonal assays: the turbidity reduction assay (TRA) (Figures 4 to 6) and the quantitative killing assay (qKA) (Figures 7 and 8). A concentration range of four concentrations was tested in both assays to demonstrate dose-dependent killing of staphylococci. Four clinically isolated strains of the two most common pathogens of the genus *Staphylococcus, S. aureus* and *S. epidermidis* were used in all assays. To estimate the activity of constructs under physiological conditions, we performed all assays in human serum and additionally compared the results of the TRAs to activities measured in PBS-T. In TRAs we measured the decrease of turbidity over time, evaluated by analyzing the area under the lysis curves and normalized the inverse area to the control. In qKAs we measured the decrease in colony forming units (CFU) over a time interval of 30 minutes and expressed the results as the logarithmic reduction in CFU numbers.

The stability of endolysins can be a limiting factor for their storage and their potency against staphylococci in the blood stream. Therefore, we also employed two assays to assess the stability of the constructs. The stability assay based on TRA data (SA-T) and the stability assay based on qKAs data (SA-Q), both include a four-hour incubation in human serum at 37°C. In these assays, the activities before and after the incubation were compared to each other. For SA-Ts the relative remaining activity was calculated (Figure 6) and for SA-Qs the CFU reduction was plotted side-by-side for both time points (Figures 7 and 8).

Overall, we observed higher activity and/or stability for the double EAD constructs with an M23 domain and an additional CHAPGH15 or a CHAPSEP domain, compared to the constructs with a single EAD: M23-LST(L)_SH3b2638 and CHAPTw(L)_SH3b2638.

### References

Grundling, A., Missiakas, D.M. & Schneewind, O., 2006. Staphylococcus aureus Mutants with Increased Lysostaphin Resistance. Journal of Bacteriology, 188(17), pp.6286-6297.
Kashani, H. et al., 2017. Recombinant Endolysins as Potential Therapeutics against AntibioticResistant Staphylococcus aureus: Current Status of Research and Novel Delivery Strategies. Clinical Microbiology Reviews, 31(1).
Korndörfer, I.P. et al., 2006. The Crystal Structure of the Bacteriophage PSA Endolysin Reveals a Unique Fold Responsible for Specific Recognition of Listeria Cell Walls. Journal of Molecular Biology, 364(4), pp.678-689.
Hilderman, R. H. Et al., 1973. Edman Degradation on In Vitro Biosynthesized Peptidoglycans from Staphylococcus epidermidis.. Journal of Bacteriology, 115(2), pp. 476-479.
Schmelcher, M. et al., 2015. Evolutionarily distinct bacteriophage endolysins featuring conserved peptidoglycan cleavage sites protect mice from MRSA infection. Journal of Antimicrobial Chemotherapy, 70(5), pp.1453-1465.
Schmelcher, M., Donovan, D.M. & Loessner, M.J., 2012. Bacteriophage endolysins as novel antimicrobials. Future Microbiology, 7(10), pp.1147-1171.
Tong, S.Y.C. et al., 2015. Staphylococcus aureus Infections: Epidemiology, Pathophysiology, Clinical Manifestations, and Management. Clinical Microbiology Reviews, 28(3), pp.603-661.
Verbree, C.T. et al., 2018. Corrected and republished from: Identification of peptidoglycan hydrolase constructs with synergistic staphylolytic activity in cow's milk. Appl Environ Microbiol 84:e02134-17.

## Claims

1. An endolysin polypeptide that has lytic activity for *Staphylococcus,* wherein the endolysin polypeptide has enhanced specific activity for *Staphylococcus epidermidis* and/or enhanced stability in human serum at 37°C compared to the endolysin with the amino acid sequence as set forward in SEQ ID NO: 4, and wherein the amino acid sequence of the endolysin polypeptide has at least 90% sequence identity with SEQ ID NO: 35.

2. A polynucleotide encoding an endolysin polypeptide according to claim 1.

3. A nucleic acid construct comprising a polynucleotide according to claim 2.

4. An expression vector comprising a nucleic acid construct according to claim 3.

5. A host cell comprising a polynucleotide according to claim 2, a nucleic acid construct according to claim 3 or an expression construct according to claim 4.

6. A composition comprising an endolysin polypeptide according to claim 1 or a polynucleotide according to claim 2, or a nucleic acid construct according to claim 3, or an expression construct according to claim 4, or a host cell according to claim 5.

7. A pharmaceutical composition comprising an endolysin polypeptide according to claim 1, or a polynucleotide according to claim 2, or a nucleic acid construct according to claim 3, or an expression construct according to claim 4, or a host cell according to claim 5, said pharmaceutical composition further comprising a pharmaceutically acceptable excipient.

8. A composition according to claim 6 or 7, further comprising an additional active ingredient.

9. A composition according to claim 6, 7, or 8, for use as a medicament,

10. A composition for use according to claim 9, wherein the composition is for use as a medicament in the prevention, delay or treatment of a condition in a subject, and wherein the condition is associated with infection with a *Staphylococcus,* such as a coagulase positive- or coagulase negative *Staphylococcus,* preferably *Staphylococcus aureus* and/or *Staphylococcus epidermidis.*

11. A composition for use according to claim 10, wherein the composition is for systemic or local administration to the subject and/or wherein the condition is selected from the group consisting of bacteraemia, infective endocarditis, prosthetic joint infection, osteomyelitis, indwelling medical device infection and implanted medical device infection.

12. *In vitro* use of an endolysin polypeptide according to claim 1, or a nucleic acid construct according to claim 3, or an expression construct according to claim 4, or a host cell according to claim 5, or a composition according to claim 6, 7 or 8, as an antimicrobial, preferably as a food additive or a disinfectant, preferably for coating a medical device.

13. An *in vitro* method for coating a medical device with an endolysin polypeptide according to claim 1, or a composition according to claim 6, 7 or 8, comprising contacting the medical device with an endolysin polypeptide according to claim 1 or a composition according to claim 6, 7 or 8.

## Patentansprüche

1. Endolysin-Polypeptid, das eine lytische Aktivität für *Staphylococcus* aufweist, wobei das Endolysin-Polypeptid eine erhöhte spezifische Aktivität für *Staphylococcus epidermidis* und/oder eine erhöhte Stabilität in menschlichem Serum bei 37 °C im Vergleich zu dem Endolysin mit der in SEQ ID NO: 4 angegebenen Aminosäuresequenz aufweist, und wobei die Aminosäuresequenz des Endolysin-Polypeptids mindestens 90 % Sequenzidentität mit SEQ ID NO: 35 aufweist.

2. Polynukleotid, das ein Endolysin-Polypeptid nach Anspruch 1 codiert.

3. Nukleinsäurekonstrukt, das ein Polynukleotid nach Anspruch 2 umfasst.

4. Expressionsvektor, der ein Nukleinsäurekonstrukt nach Anspruch 3 umfasst.

5. Wirtszelle, die ein Polynukleotid nach Anspruch 2, ein Nukleinsäurekonstrukt nach Anspruch 3 oder ein Expressionskonstrukt nach Anspruch 4 umfasst.

6. Zusammensetzung, die ein Endolysin-Polypeptid nach Anspruch 1 oder ein Polynukleotid nach Anspruch 2 oder ein Nukleinsäurekonstrukt nach Anspruch 3 oder ein Expressionskonstrukt nach Anspruch 4 oder eine Wirtszelle nach Anspruch 5 umfasst.

7. Pharmazeutische Zusammensetzung, die ein Endolysin-Polypeptid nach Anspruch 1 oder ein Polynukleotid nach Anspruch 2 oder ein Nukleinsäurekonstrukt nach Anspruch 3 oder ein Expressionskonstrukt nach Anspruch 4 oder eine Wirtszelle nach Anspruch 5 umfasst, wobei die pharmazeutische Zusammensetzung ferner einen pharmazeutisch akzeptablen Hilfsstoff umfasst.

8. Zusammensetzung nach Anspruch 6 oder 7, die ferner einen zusätzlichen Wirkstoff umfasst.

9. Zusammensetzung nach Anspruch 6, 7 oder 8 zur Verwendung als Arzneimittel.

10. Zusammensetzung zur Verwendung nach Anspruch 9, wobei die Zusammensetzung zur Verwendung als Arzneimittel zur Vorbeugung, Verzögerung oder Behandlung einer Erkrankung bei einem Patienten bestimmt ist und wobei die Erkrankung mit einer Infektion mit einem *Staphylococcus,* wie beispielsweise einem koagulase-positiven oder koagulase-negativen *Staphylococcus,* vorzugsweise *Staphylococcus aureus* und/oder *Staphylococcus epidermidis,* assoziiert ist.

11. Zusammensetzung zur Verwendung nach Anspruch 10, wobei die Zusammensetzung zur systemischen oder lokalen Verabreichung an den Patienten bestimmt ist und/oder wobei die Erkrankung aus der Gruppe ausgewählt ist, die aus Bakteriämie, infektiöser Endokarditis, Infektion einer Gelenkprothese, Osteomyelitis, Infektion durch eine medizinische Verweilvorrichtung und Infektion durch eine implantierte medizinische Vorrichtung besteht.

12. In-vitro-Verwendung eines Endolysin-Polypeptids nach Anspruch 1 oder eines Nukleinsäurekonstrukts nach Anspruch 3 oder eines Expressionskonstrukts nach Anspruch 4 oder einer Wirtszelle nach Anspruch 5 oder einer Zusammensetzung nach Anspruch 6, 7 oder 8 als antimikrobielles Mittel, vorzugsweise als Lebensmittelzusatzstoff oder Desinfektionsmittel, vorzugsweise zum Beschichten einer medizinischen Vorrichtung.

13. *In-vitro-*Verfahren zum Beschichten einer medizinischen Vorrichtung mit einem Endolysin-Polypeptid nach Anspruch 1 oder mit einer Zusammensetzung nach Anspruch 6, 7 oder 8, umfassend das Inkontaktbringen der medizinischen Vorrichtung mit einem Endolysin-Polypeptid nach Anspruch 1 oder einer Zusammensetzung nach Anspruch 6, 7 oder 8.

## Revendications

1. Polypeptide endolysine qui a une activité lytique sur *Staphylococcus,* où le polypeptide endolysine a une activité spécifique renforcée sur *Staphylococcus epidermidis* et/ou une stabilité renforcée dans le sérum humain à 37 °C par rapport à l'endolysine ayant la séquence d'acides aminés telle que présentée dans la SEQ ID NO : 4, et où la séquence d'acides aminés du polypeptide endolysine a au moins 90 % d'identité de séquence avec la SEQ ID NO : 35.

2. Polynucléotide codant pour un polypeptide endolysine selon la revendication 1.

3. Construction d'acide nucléique comprenant un polynucléotide selon la revendication 2.

4. Vecteur d'expression comprenant une construction d'acide nucléique selon la revendication 3.

5. Cellule hôte comprenant un polynucléotide selon la revendication 2, une construction d'acide nucléique selon la revendication 3 ou une construction d'expression selon la revendication 4.

6. Composition comprenant un polypeptide endolysine selon la revendication 1 ou un polynucléotide selon la revendication 2, ou une construction d'acide nucléique selon la revendication 3, ou une construction d'expression selon la revendication 4, ou une cellule hôte selon la revendication 5.

7. Composition pharmaceutique comprenant un polypeptide endolysine selon la revendication 1, ou un polynucléotide selon la revendication 2, ou une construction d'acide nucléique selon la revendication 3, ou une construction d'expression selon la revendication 4, ou une cellule hôte selon la revendication 5, ladite composition pharmaceutique comprenant en outre un excipient pharmaceutiquement acceptable.

8. Composition selon la revendication 6 ou 7, comprenant en outre un ingrédient actif supplémentaire.

9. Composition selon la revendication 6, 7 ou 8, pour utilisation comme médicament.

10. Composition pour utilisation selon la revendication 9, où la composition est destinée à être utilisée comme médicament dans la prévention, le retardement ou le traitement d'une affection chez un sujet, et où l'affection est associée à une infection par un *Staphylococcus,* tel qu'un *Staphylococcus* à coagulase positive ou à coagulase négative, de préférence *Staphylococcus aureus* et/ou *Staphylococcus epidermidis.*

11. Composition pour utilisation selon la revendication 10, où la composition est destinée à être administrée par voie systémique ou locale au sujet et/ou où l'affection est choisie dans le groupe constitué par la bactériémie, l'endocardite infectieuse, l'infection d'une prothèse articulaire, l'ostéomyélite, l'infection d'un dispositif médical à demeure et l'infection d'un dispositif médical implanté.

12. Utilisation *in vitro* d'un polypeptide endolysine selon la revendication 1, ou d'une construction d'acide nucléique selon la revendication 3, ou d'une construction d'expression selon la revendication 4, ou d'une cellule hôte selon la revendication 5, ou d'une composition selon la revendication 6, 7 ou 8, comme antimicrobien, de préférence comme additif alimentaire ou désinfectant, de préférence pour le revêtement d'un dispositif médical.

13. Procédé *in vitro* pour revêtir un dispositif médical d'un polypeptide endolysine selon la revendication 1, ou d'une composition selon les revendications 6, 7 ou 8, comprenant la mise en contact du dispositif médical avec un polypeptide endolysine selon la revendication 1 ou avec une composition selon les revendications 6, 7 ou 8.
